# EUROPEAN PATENT SPECIFICATION

(11) **EP 3 931 346 B1**
(45) Date of publication and mention of the grant of the patent: **25.02.2026**
(21) Application number: 20763826.3
(22) Date of filing: 26.02.2020
(51) Int. Cl.: C12Q 1/26, C12Q 1/32, C12Q 1/66, C12Q 1/6813, C12Q 1/6816, C12Q 1/6827

(54) **USING TETHERED ENZYMES TO DETECT NUCLEIC ACIDS**
VERWENDUNG VON GEBUNDENEN ENZYMEN ZUR DETEKTION VON NUKLEINSÄUREN
UTILISATION D'ENZYMES ATTACHÉES POUR DÉTECTER DES ACIDES NUCLÉIQUES

(30) Priority: 26.02.2019 US 201962810448 P
(43) Date of publication of application: 05.01.2022
(73) Proprietor: Cornell University, Ithaca, NY 14850 (US)
(72) Inventor: COHEN, Roy, Ithaca, NY 14850 (US); TRAVIS, Alexander, Ithaca, NY 14850 (US)
(74) Representative: Potter Clarkson
(86) International application number: PCT/US2020/019924
(87) International publication number: WO 2020/176638

(56) References cited:
- CN-A- 106 868 223
- US-A1- 2003 124 544
- US-A1- 2003 207 295
- US-A1- 2004 197 845
- US-A1- 2005 142 589
- US-A1- 2006 003 322
- US-A1- 2009 317 803
- US-A1- 2015 118 218
- US-A1- 2018 136 203
- COHEN ROY ET AL: "Use of Tethered Enzymes as a Platform Technology for Rapid Analyte Detection", PLOS ONE, vol. 10, no. 11, 25 November 2015 (2015-11-25), pages e0142326, XP055965964, DOI: 10.1371/journal.pone.0142326
- CHINATSU MUKAI ET AL: "Biomimicry Promotes the Efficiency of a 10-Step Sequential Enzymatic Reaction on Nanoparticles, Converting Glucose to Lactate", ANGEWANDTE CHEMIE INTERNATIONAL EDITION, vol. 56, no. 1, 30 November 2016 (2016-11-30), pages 235 - 238, XP055733879, ISSN: 1433-7851, DOI: 10.1002/anie.201609495

## Description

This application claims the benefit of U.S. Provisional Patent Application Serial No. 62/810,448, filed February 26, 2019.

### FIELD

The present application relates to the use of tethered enzymes to detect nucleic acids.

### BACKGROUND

Nucleic acid amplification may be used to determine whether a particular template nucleic acid is present in a sample. If an amplification product is produced, this indicates that the template nucleic acid was present in the sample. Conversely, the absence of any amplification product indicates the absence of template nucleic acid in the sample. Such techniques are of great importance in diagnostic applications, for example, for determining whether a pathogen is present in a sample.

Nucleic acids may be amplified by a variety of thermocycling and isothermal techniques. Thermocycling techniques, such as the polymerase chain reaction (PCR), use temperature cycling to drive repeated cycles of DNA synthesis leading to large amounts of new DNA being synthesised in proportion to the original amount of template DNA. Recently, a number of isothermal techniques have also been developed that do not rely on thermocycling to drive the amplification reaction. Isothermal techniques which utilize DNA polymerases with strand-displacement activity have been developed for amplification reactions that do not involve an RNA-synthesis step. Similarly, for amplification reactions that do involve an RNA-synthesis step, isothermal techniques have been developed that use reverse transcriptase, RNase H, and a DNA-dependent RNA polymerase.

Nonetheless, the detection and/or quantitation of specific nucleic acid sequences is an important technique for identifying and classifying microorganisms, diagnosing infectious diseases, detecting and characterizing genetic abnormalities, identifying genetic changes associated with cancer, studying genetic susceptibility to disease, and measuring response to various types of treatment. Such procedures are also useful in detecting and quantitating microorganisms in foodstuffs, water, industrial and environmental samples, seed stocks, and other types of material where the presence of specific microorganisms may need to be monitored. Other applications are found in the forensic sciences, anthropology, archaeology, and biology where measurement of the relatedness of nucleic acid sequences has been used to identify criminal suspects, resolve paternity disputes, construct genealogical and phylogenetic trees, and aid in classifying a variety of life forms.

Advances in the field of molecular biology over the last 20 years have allowed the detection of specific nucleic acid sequences in test samples obtained from patients and other subjects. Such test samples include serum, urine, stool, saliva, amniotic fluid, and other body fluids. Thus, a number of methods to detect and/or quantitate nucleic acid sequences are well known in the art. However, an inherent result of highly sensitive nucleic amplification systems is the emergence of side-products. Side-products include molecules which may, in some systems, interfere with the amplification reaction, thereby lowering specificity. This is because limited amplification resources, including primers and enzymes needed in the formation of primer extension and transcription products are diverted to the formation of side-products. In some situations, the appearance of side-products can also complicate the analysis of amplicon production by various molecular techniques. In addition, in many cases of interest, specific nucleic acid sequences are present at very low concentrations in the sample being tested for the required nucleic acid sequence. In such cases, if the assay sensitivity cannot be increased, the presence of the required molecule cannot be detected.
US 2009/317803 describes a technology platform for quantitation number of copies of nucleic acid molecules of interest by lumonogenic (i.e., enzymatic luminescence) detection.
Cohen Roy et al 2015 PLOS ONE 10: e0142326 discusses the use of tethered enzymes as a platform technology for rapid analyte detection.

The present application is directed to overcoming these and other deficiencies in the art.

### SUMMARY

One aspect of the present application relates to a method of detecting a target nucleic acid molecule in a sample. The method includes providing a sample containing a target nucleic acid molecule. The method includes contacting a sample with a capture oligonucleotide molecule complementary to at least a portion of the target nucleic acid molecule so that the capture oligonucleotide molecule hybridizes to a complementary portion of the target nucleotide molecule and forms a double-stranded nucleic acid molecule. The capture oligonucleotide molecule has (i) a length of 30-60 bases, (ii) a 3' tail consisting of 4-8 nucleotides , (iii) a 5' tail, (iv) a target-specific portion between the 3' tail and the 5' tail, (v) a deoxy-adenosine phosphate content of 40-50%, (vi) no deoxy thymidine phosphate in the 3' tail or the 5' tail, and (vii) the 3' tail and the 5' tail collectively comprising a deoxyadenosine phosphate content which is 40-50% of said length of 30-60 bases of the capture oligonucleotide molecule. The double-stranded nucleic acid molecule, a polymerase, and a dNTP mixture are contacted together to form a polymerase extension mixture, wherein deoxy-adenosine triphosphate is excluded from the dNTP mixture. The polymerase extension mixture is subjected to conditions under which the target nucleic acid molecule is extended isothermally and releases free phosphates. Adenosine triphosphates are then produced from the released free phosphates, and the adenosine triphosphates produced from the free phosphates are metabolized with a luciferase to produce a bioluminescent readout signal, indicating the presence of the target nucleic acid molecule in the sample.

Another aspect of the present application relates to a method of detecting a target nucleic acid molecule in a sample. The method includes providing a sample containing a target nucleic acid molecule. The method includes contacting the sample with a capture oligonucleotide molecule complementary to at least a portion of the target nucleic acid molecule so that the capture oligonucleotide molecule hybridizes to a complementary portion of the target nucleic acid molecule and forms a double-stranded nucleic acid molecule, wherein said capture oligonucleotide has:
a) a length of 30-60 bases;
b) a 3' tail consisting of 4 to 8 nucleotides, optionally has a 5' tail, optionally (i) a target-specific portion between the 3' tail and the 5' tail; or (ii) no deoxy thymidine phosphate in the 3' tail or the 5' tail; or
c) a 3' tail and a 5' tail collectively comprising a deoxy-adenosine phosphate content which is 40-50% of said length of 30-60 bases of the capture oligonucleotide molecule.
The double-stranded nucleic acid molecule, a polymerase, and a dNTP mixture are contacted together to form a polymerase extension mixture, wherein deoxy-adenosine triphosphate is excluded from the dNTP mixture. The polymerase extension mixture is subjected to conditions under which the target nucleic acid molecule is extended isothermally and releases free phosphates. Adenosine triphosphates are then produced enzymatically from the released free phosphates, and the adenosine triphosphates produced from the free phosphates are metabolized with a luciferase to produce a bioluminescent readout signal, indicating the presence of the target nucleic acid molecule in the sample. The DNA polymerase, the luciferase, and the enzyme producing adenosine triphosphates are each coupled to a solid support.

Another aspect of the present application relates to a kit for detecting a target nucleic acid molecule in a sample. The kit includes a capture oligonucleotide molecule complementary to at least a portion of the target nucleic acid molecule so that the capture oligonucleotide molecule hybridizes to a complementary portion of the target nucleic acid molecule and forms a double-stranded nucleic acid molecule, wherein said capture oligonucleotide molecule has (i) a length of 30-60 bases, (ii) a 3' tail consisting of 4 to 8 nucleotides, (iii) a 5' tail, (iv) a target-specific portion between the 3' tail and the 5' tail, (v) a deoxy-adenosine phosphate content of 40-50%, (vi) no deoxy thymidine phosphate in the 3' tail or the 5' tail, and (vii) the 3' tail and the 5' tail collectively comprising a deoxy-adenosine phosphate content which is 40-50% of that of said length of 30-60 bases of the capture oligonucleotide molecule, a polymerase coupled to a solid support; a dNTP mixture, wherein deoxy-adenosine triphosphate is excluded from the dNTP mixture, an enzyme for producing adenosine triphosphates from released free phosphates coupled to a solid support, and a luciferase for producing a bioluminescent readout signal, where the luciferase is coupled to a solid support.

Another aspect of the present application relates to a kit for detecting a target nucleic acid molecule in a sample. The kit includes a capture oligonucleotide molecule complementary to at least a portion of the target nucleic acid molecule so that the capture oligonucleotide molecule hybridizes to a complementary portion of the target nucleic acid molecule and forms a double-stranded nucleic acid molecule. The capture oligonucleotide molecule has (i) a length of 30-60 bases, (ii) a 3' tail consisting of 4 to 8 nucleotides, (iii) a 5' tail, (iv) a target-specific portion between the 3' tail and the 5' tail, (v) a deoxy-adenosine phosphate content of 40-50%, (vi) no deoxy thymidine phosphate in the 3' tail or the 5' tail, and (vii) the 3' tail and the 5' tail collectively comprising a deoxy-adenosine phosphate content which is 40-50% of that of said length of 30-60 bases of the capture oligonucleotide molecule. The kit also includes a polymerase, a dNTP mixture, wherein deoxy-adenosine triphosphate is excluded from the dNTP mixture, an enzyme for producing adenosine triphosphates from released free phosphates, and a luciferase for producing a bioluminescent readout signal.

A final aspect of the present application relates to the use of a composition in the methods of the invention that comprises a capture oligonucleotide molecule, wherein the capture oligonucleotide molecule has (i) a length of 30-60 bases, (ii) a 3' tail consisting of 4 to 8 nucleotides, (iii) a 5' tail, (iv) a target-specific portion between the 3' tail and the 5' tail, (v) a deoxy-adenosine phosphate content of 40-50%, (vi) no deoxy thymidine phosphate in the 3' tail or the 5' tail, and (vii) the 3' tail and the 5' tail collectively comprise deoxy-adenosine phosphate content which is 40-50% of said length of 30-60 bases of the capture oligonucleotide molecule.

The present application discloses significant advances in methods of detecting nucleic acids through the use of, for example, enzyme reactions in which the enzymes are tethered to surfaces (e.g., nanoparticles). The assays described herein are transduced into a common luminescent output. That is, in certain embodiments, they may be all linked to bioluminescent (BL) proteins or substrates that will allow light to be emitted and read, with the amount of that light correlated to the amount of target nucleic acid in the system or biological sample. This technology is suitable for generating qualitative as well as quantitative results for various nucleic acid molecules.

The present application confers multiple advantages over other detection methods and systems. These include: 1) speed - assays using enzymatic reactions occur quickly, providing readout within several minutes; 2) luminescence-based readouts are used, which enable stand-alone, highly portable systems and devices that do not require bulky excitation elements (such as those needed for fluorescence); 3) sensitivity - due to release of multiple free phosphates for each hybridization event and enzymatic reaction assays facilitating signal amplification at the steps of both detection and readout; 4) reduced cost of fabrication -- likely components of such systems, including, e.g., nanoparticles, may be made from inexpensive materials and can easily be mass produced; 5) multiplex capability - coupled biochemical reactions could detect multiple nucleic acid molecules in a single system in certain embodiments of the present application; 6) use of tethered enzymes facilitates maximum enzyme stability and activity; 7) use of tethered enzymes confines reactions and readouts to specific areas of the system (e.g., a specific region of a card), reducing the size of a photodetector in the reader; 8) use of tethered enzymes confines reactions and readouts allowing for in-line negative controls and controls for background luminescence; 9) use of tethered enzymes confines reactions and readouts, reducing light contamination from detection of other nucleic acid molecules in the same system; 10) immobilization of the capture-oligonucleotide enhances the ability to detect multiple target-oligonucleotides in specific areas of the system (e.g., a specific region of a card); 11) using isothermal amplification enables detection in ambient temperature without the need of temperature cycling; and 12) the design of the capture oligonucleotide enables a single-step reaction with no interference/inhibition of side-products, and the ability to incorporate the bioluminescent enzyme into the single-step reaction enables an additional level of signal amplification coming from the release of free phosphates from the metabolized AP molecules, which feed back into the ATP-generating reaction .

### BRIEF DESCRIPTION OF THE DRAWINGS

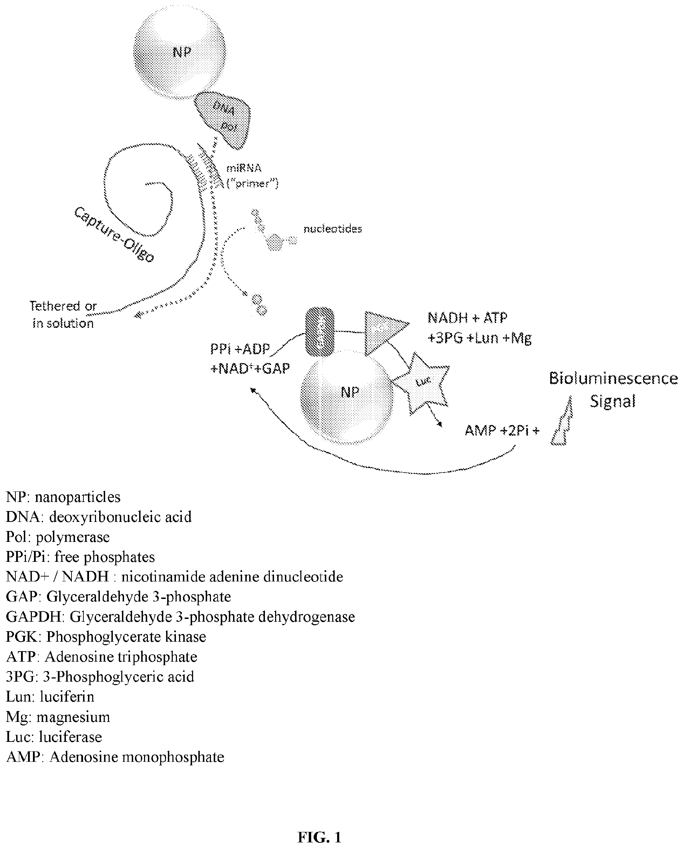

FIG. 1 shows an embodiment of the tethered enzyme technology (TET)-miRNA reaction. miRNA (or other single-strand nucleic acid polymers) anneals to the complementary insert sequence within the Capture-Oligonucleotide and generates a double strand. This allows the DNA-polymerase to bind and initiate the polymerization reaction, which releases free phosphates (PPi) through nucleotide incorporation (isothermal replication). In the presence of ADP, NAD+ and GAP, these PPi groups are then used by the coupled GAPDH-PGK enzymatic reaction to generate ATP. Finally, luciferase hydrolyses the ATP to generate a bioluminescence signal.
FIG. 2 shows another embodiment of the TET-miRNA reaction. miRNA (or other single-strand nucleic acid polymers) anneals to the complementary insert sequence within the capture-oligonucleotide and generates a double strand. This allows the DNA-polymerase to bind and initiate the polymerization reaction, which releases free phosphates (PPi) through nucleotide incorporation (isothermal replication). In the presence of APS, these PPi groups are then used by the enzyme ATP-sulfurylase to generate ATP. Finally, luciferase hydrolyses the ATP to generate a bioluminescence signal.
FIG. 3 shows capture oligonucleotide (Cap-Oligo) design and features.
FIG. 4 shows that excluding dATP from the reaction mixture improves assay kinetics and sensitivity.
FIGs. 5A-5D show the TET-miRNA assay can detect mismatching nucleotides in the 3' end of the target oligonucleotide, as well as various analysis or quantification of data.
FIGs. 6A-6B show testing of the TET-miRNA assay range of sensitivity for target-oligonucleotide detection.
FIGS. 7A-7C show that the design of 5' and 3' tails of the capture-oligonucleotide affects TET-miRNA reaction kinetics.
FIGs. 8A-8B show capture-oligonucleotide dATP content affects TET-miRNA reaction kinetics.
FIGs. 9A-9B show 3' tail length of the capture-oligonucleotide affects the TET-miRNA reaction activity and kinetics.
FIGS. 10A-10C show the TET-miRNA assay is sensitive to mutations in the sequence of MIR-340 target-oligonucleotide (lung cancer related microRNA).
FIGs. 11A-11B show the TET-miRNA assay can detect naturally occurring miRNA in human serum.
FIG. 12 shows the TET-miRNA assay can detect naturally occurring miRNA in human plasma.
FIGs. 13A-13B show ribonuclease (RNAse) treatment abolishes the TET-miRNAs activity.
FIGs. 14A-14B show target-oligonucleotide detection with (non-oriented) NP-immobilized vs. in-solution reactions.
FIGs. 15A-15B show immobilization of commercial BST2.0 onto NPs via biotin-streptavidin binding inhibits the TET-miRNA reaction assay.
FIGs. 16A-16C show the TET-miRNA assay is only marginally affected by temperature.
FIGs. 17A-17E show the testing of two different capture-oligonucleotide designs for detecting miRNA in human serum (non-tethered).
FIGs. 18A-18B show a comparison of the activity of various DNA polymerase's activity in the TET-miRNA assay.
FIGs. 19A-19D show target-oligonucleotide detection using TET-miRNA when tethered versus in solution.
FIGs. 20A-20C show a comparison of His-Si-Klenow versus His-Si-BST activity in the TET-miRNA assay - tethered versus non-tethered.
FIGs. 21A-21B show a comparison of target-oligonucleotide detection using various ratios of His-Si-ATPS/His-Si-BST/NPs.
FIGs. 22A-22C show miRNA (RNA oligo) detection using TET-miRNA (His-Si-enzymes) and DNA capture-oligonucleotide.
FIGs. 23A-23C show testing the target-oligonucleotide mismatch sensitivity of different non-tethered DNA polymerases (His-Si-Klenow vs. His-Si-BST).
FIG. 24 shows testing the TET-miRNA reaction when the capture-oligonucleotide is in solution versus immobilized (non-oriented).
FIGs. 25A-25B show testing the TET-miRNA reaction when the capture-oligonucleotide is in solution versus immobilized via biotin-streptavidin to SiO₂ NPs.

### DETAILED DESCRIPTION

One aspect of the present application relates to a method of detecting a target nucleic acid molecule in a sample. The method includes providing a sample containing a target nucleic acid molecule. The method includes contacting the sample with a capture oligonucleotide molecule complementary to at least a portion of the target nucleic acid molecule so that the capture oligonucleotide molecule hybridizes to a complementary portion of the target nucleotide molecule and forms a double-stranded nucleic acid molecule. The capture oligonucleotide molecule has (i) a length of 30-60 base pairs, (ii) a 4-8 base pair overhang on its 3' end, (iii) a 5' tail, (iv) a target-specific portion between the 3' end and the 5' tail, (v) a deoxy-adenosine diphosphate content of 40-50%, (vi) no deoxy thymidine phosphate in the 3' end or the 5' tail, and (vii) the 3' end and the 5' tail having an ATP content which is 40-50% of that of the capture oligonucleotide molecule. The double-stranded nucleic acid molecule, a polymerase, and a dNTP mixture are contacted together to form a polymerase extension mixture, wherein deoxy-adenosine triphosphate is excluded from the dNTP mixture. The polymerase extension mixture is subjected to conditions under which the target nucleic acid molecule is extended isothermally and releases free phosphates. Adenosine triphosphates are then produced from the released free phosphates, and the adenosine triphosphates produced from the free phosphates are metabolized with a luciferase to produce a bioluminescent readout signal, indicating the presence of the target nucleic acid molecule in the sample.
Another aspect of the present application relates to a method of detecting a target nucleic acid molecule in a sample. The method includes providing a sample containing a target nucleic acid molecule. The method includes contacting the sample with a capture oligonucleotide molecule complementary to at least a portion of the target nucleic acid molecule so that the capture oligonucleotide molecule hybridizes to a complementary portion of the target nucleic acid molecule and forms a double-stranded nucleic acid molecule. The capture oligonucleotide has
a) a length of 30-60 bases;
b) a 3' tail consisting of 4 to 8 nucleotides, optionally has a 5' tail, optionally (i) a target-specific portion between the 3' tail and the 5' tail; or (ii) no deoxy thymidine phosphate in the 3' tail or the 5' tail; or
c) a 3' tail and a 5' tail collectively comprising a deoxy-adenosine phosphate content which is 40-50% of said length of 30-60 bases of the capture oligonucleotide molecule.
The double-stranded nucleic acid molecule, a polymerase, and a dNTP mixture are contacted together to form a polymerase extension mixture, wherein deoxy-adenosine triphosphate is excluded from the dNTP mixture. The polymerase extension mixture is subjected to conditions under which the target nucleic acid molecule is extended isothermally and releases free phosphates. Adenosine triphosphates are then produced enzymatically from the released free phosphates, and the adenosine triphosphates produced from the free phosphates is metabolized with a luciferase to produce a bioluminescent readout signal, indicating the presence of the target nucleic acid molecule in the sample. The DNA polymerase, the luciferase, and the enzyme producing adenosine triphosphates are each coupled to a solid support.

As shown in FIG. 1, DNA polymerase is tethered to one nanoparticle, and GAPDH, PGK, and luciferase ("Luc") are tethered to another nanoparticle. An miRNA target anneals to the complementary insert sequence within a provided capture-oligonucleotide, either tethered or in solution, and a double strand is generated. This allows the DNA polymerase to bind and initiate the polymerization reaction, thereby releasing free phosphates (PPi) through nucleotide incorporation. ADP, NAD+, and glyceraldehyde 3-phosphate are provided for the assay. In the presence of these components, the free phosphates are used by the tethered GAPDH and PGK to generate ATP. In conjunction with luciferin, the ATP will then be used by tethered Luc to generate a bioluminescent signal. The amount of light emitted is directly proportional to the amount of ATP in the system, thereby corresponding to the amount of target miRNA in the system. Light emitted may, in one embodiment, be read quantitatively and/or qualitatively by a photodetector positioned to capture that emitted signal.

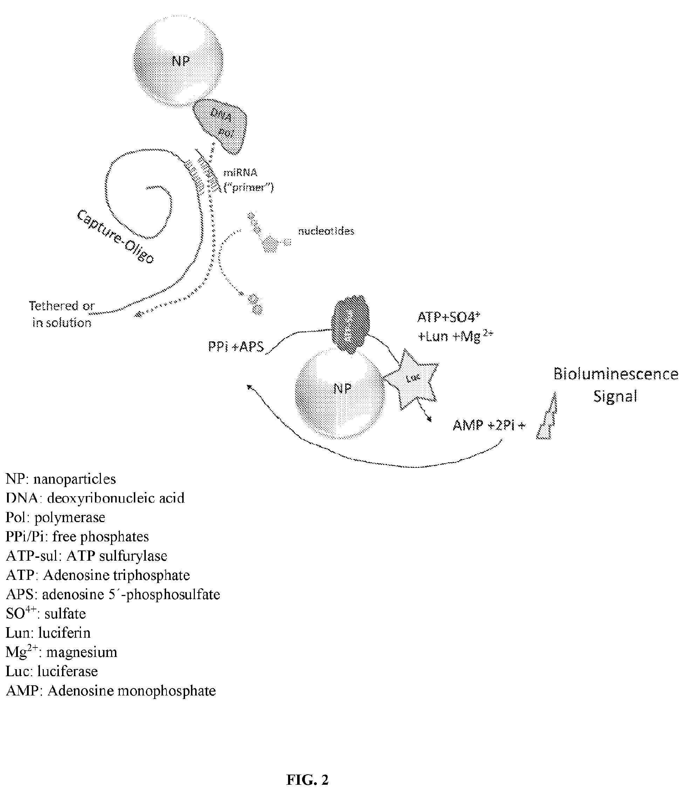

Alternatively, as shown in FIG. 2, DNA polymerase is tethered to one nanoparticle, and ATP sulfurylase (ATP-sul) and luciferase ("Luc") are tethered to another nanoparticle. An miRNA target anneals to the complementary insert sequence within a provided capture-oligonucleotide, either tethered or in solution, and a double strand is generated. This allows the DNA polymerase to bind and initiate the polymerization reaction, thereby releasing free phosphates (PPi) through nucleotide incorporation. Adenosine 5'-phosphosulfate (APS) is provided for the assay. In the presence of APS, the free phosphates are used by the tethered ATP-sul to generate ATP. In conjunction with luciferin, the ATP will then be used by tethered Luc to generate a bioluminescent signal. The amount of light emitted is directly proportional to the amount of ATP in the system, thereby corresponding to the amount of target miRNA in the system. Light emitted may, in one embodiment, be read quantitatively and/or qualitatively by a photodetector positioned to capture that emitted signal.

Suitable biological samples in accordance with the present application include biological samples including, but not limited to, blood, blood serum, blood plasma, cerebrospinal fluid, urine, saliva, tissue. Industrial samples can include food, beverages, and synthetic materials. Environmental samples can include water, air, or surface samples.

The term "nucleic acid" refers to polymers of nucleotides (e.g., ribonucleotides and deoxyribonucleotides, both natural and non-natural) including DNA, RNA, and their subcategories, such as cDNA, mRNA, miRNA etc. A nucleic acid may be single-stranded and will generally contain 5'-3' phosphodiester bonds, although in some cases, nucleotide analogs may have other linkages. Nucleic acids may include naturally occurring bases (adenosine, guanosine, cytosine, uracil and thymidine) as well as non-natural bases.

As used herein, the terms "target nucleic acid" or "target" refer to a portion of the nucleic acid sequence in the sample which is to be detected or analyzed. The term target includes all variants of the target sequence, e.g., one or more mutant variants and the wild type variant.

In one embodiment, the target nucleic acid molecule is micro-RNA.

As used herein, a "capture oligonucleotide" refers to a nucleic acid fragment that specifically hybridizes to a target sequence in a target nucleic acid by standard base pairing. As used herein, "specifically hybridize" is meant that under stringent hybridization assay conditions, capture oligonucleotides hybridize to their target sequences, or replicates thereof, to form stable capture oligonucleotides:target hybrids, while at the same time formation of stable capture oligonucleotides:non-target hybrids is minimized. Thus, a capture oligonucleotides hybridizes to a target sequence or replicate thereof to a sufficiently greater extent than to a non-target sequence. Appropriate hybridization conditions are well-known in the art, may be predicted based on sequence composition, or can be determined by using routine testing methods (see, e.g., Sambrook et al., Molecular Cloning, A Laboratory Manual, 2nd ed. (Cold Spring Harbor Laboratory Press, Cold Spring Harbor, N.Y., 1989).

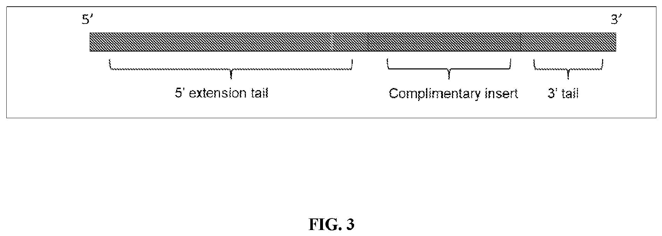

FIG. 3 depicts an embodiment of the design of the capture oligonucleotide. As shown in FIG. 3, the capture oligonucleotide may be 30-60 nucleotides in length including a 5' extension tail, an internal complementary insert, and a 3' tail. The 5' extension tail and the 3' tail sequences do not contain dTTP. The ATP content of the 5' extension tail and the 3' tail is 40-50% of the entire oligonucleotide. The optimal capture oligonucleotide sequence includes four to eight additional nucleotides on the 3' tail. Preferably, the capture oligonucleotide is designed to not hybridize with itself to form a hairpin structure in such a way as to interfere with hybridization to the target nucleic acid.

As used herein, "luciferase" refers to an oxygenase that catalyzes a luminescence reaction as follows:

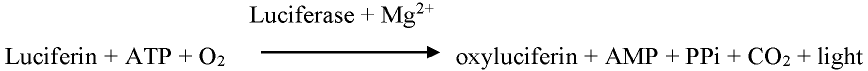

Thus, luciferase refers to an enzyme or photoprotein that catalyzes a bioluminescent reaction (a reaction that produces bioluminescence) and is a naturally occurring, recombinant, or mutated luciferase unless otherwise specified. When present in nature, luciferase can be readily obtained from an organism by one of ordinary skill in the art. If the luciferase is a naturally occurring luciferase, or a recombinant or mutant luciferase (*e.g,* a luciferase that retains activity in a luciferase-luciferin reaction of a naturally occurring luciferase), the nucleic acid encoding the luciferase is expressed. It can be easily obtained from cultures of bacteria, yeast, mammalian cells, insect cells, plant cells and the like transformed into. Furthermore, recombinant or mutant luciferases can be easily obtained from *in vitro* cell-free systems that use nucleic acids encoding luciferases. Luciferase is available from Promega Corporation, Madison, WI.. Luciferases, modified mutants or variants thereof are also known in the art and described in, for example, Thorne et al, "Illuminating Insights into Firefly Luciferase and Other Bioluminescent Reporters Used in Chemical Biology," Chemistry & Biology 17(6):646-657 (2010).

The "polymerase extension" reaction according to the application includes all forms of template-directed polymerase catalyzed nucleic acid synthesis reactions. Conditions and reagents for primer extension reactions are known in the art, and any of the standard methods, reagents and enzymes, etc. can be used at this stage (*see,* for example, Sambrook et al ., (editors), Molecular Cloning: a Laboratory Manual (1989), Cold Spring Harbor Laboratory Press). Thus, the extension reaction in its most basic form is performed in the presence of the primer, deoxynucleotides (dNTP) and a suitable polymerase enzyme, for example, Klenow, or indeed any available and suitable enzyme polymerase. By way of example, polymerases suitable for use in the methods of the present application are well known in the art and include, without limitation, full length BST DNA polymerase, large fragment BST DNA polymerase, BST 2.0 DNA Polymerase, Klenow fragment (3' to 5' exo), and DNA Polymerase I (large Klenow fragment). The conditions can be selected according to the choice, according to the procedures known in the art.

Polymerase extension techniques for use in the methods of the present application are isothermal techniques (*i.e.,* those that are performed at a single temperature or where the major aspect of the amplification process is performed at a single temperature). Such techniques rely on the ability of a polymerase to copy the template strand being amplified to form a bound duplex. The isothermal techniques rely on a strand displacing polymerase in order to separate/displace the two strands of the duplex and re-copy the template. This well-known property has been the subject of numerous scientific articles (*see* for example Y. Masamute et al., J. Biol. Chem. 246:2692-2701 (1971); R. L. Lechner et al., J. Biol. Chem. 258:11174-11184 (1983); and R. C. Lundquist and B. M. Olivera, Cell 31:53-60 (1982)).

Briefly, as used in the methods of the present application, the polymerase extension occurs when DNA polymerase binds to a capture oligonucleotide-target hybrid (*i.e.,* double stranded DNA) and extends the complementary DNA strand based on the capture oligonucleotide sequence. The extension reaction occurs using available nucleotides provided in a deoxynucleotide (dNTP) mix added to the reaction. These dNTPs include deoxy-adenosine triphosphate (dATP), deoxy-thymidine triphosphate (dTTP), deoxy-cytidine triphosphate (dCTP), and deoxy-guanosine triphosphate (dGTP)..Deoxy-adenosine triphosphate is excluded from the polymerase extension mixture.

As described above, polymerase extension techniques for use in the methods of the present application are isothermal techniques (*i.e.,* those that are performed at a single temperature or where the major aspect of the amplification process is performed at a single temperature). Accordingly, in certain embodiments, the polymerase extension reaction is carried out at a temperature of 0, 5, 10, 15, 20, 25, 30, 35, 40, 45, 50, 55, 60, 65, 70, 75, 80, 85, 90, or 95 to 100 °C. In one embodiment, the polymerase extension reaction is carried out at a temperature of 25 to 40 °C.

The polymerase extension reaction releases two phosphate groups (PPi) per nucleotide added to the DNA strand. In the methods of the present application, the release of these free phosphates (PPi) can then be used to facilitate detection of the target nucleic acid molecule in the sample. Specifically, through the conversion of PPi to ATP via enzymatic reaction and the subsequent bioluminometric detection of ATP using the signal-transducing molecule luciferase, the presence or absence of a target nucleic acid molecule can be detected.

Luciferase and luciferin are used, in combination, to identify the target nucleic acid since the amount of light generated is substantially proportional to the amount of ATP generated, and, in turn, is directly proportional to the amount of nucleotide incorporated and target nucleic acid present. Thus, the method also includes providing luciferin and O₂, where the luciferin and O₂ are added to the reaction.

As described above, the method described herein involves subjecting a polymerase extension mixture to conditions under which the target nucleic acid molecule is extended and releases free phosphates. Adenosine triphosphates (ATP) are then produced from the released free phosphates via an enzymatic reaction, which is then metabolized with a luciferase to produce the bioluminescent readout signal. In accordance with this aspect, in one embodiment, producing adenosine triphosphates comprises subjecting the released free phosphates to a coupled glyceraldehyde 3-phosphate dehydrogenase-phosphoglycerate kinase (GAPDH-PGK) enzymatic reaction to produce adenosine triphosphate.

In this embodiment, the enzymatic reaction involves the GAPDH reacting with PGK in the presence of PPi, adenosine diphosphate (ADP), nicotinamide adenine dinucleotide (NAD+), and glyceraldehyde 3-phosphate (GAP) to produce ATP. ATP then reacts with luciferase to produce a measurable signal. The reaction scheme is shown below:

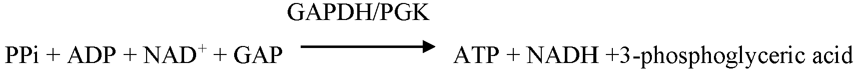

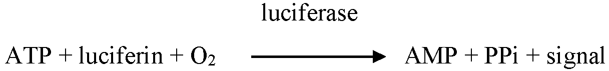

In another embodiment, adenosine triphosphates may be produced by contacting the released free phosphates with adenosine 5'-phosphosulfate (APS) in the presence of adenosine triphosphate sulfurylase (ATP-sul) to produce adenosine triphosphate. ATP then reacts with luciferase to produce a measurable signal. The reaction scheme is shown below:

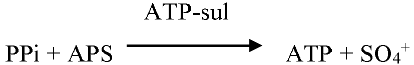

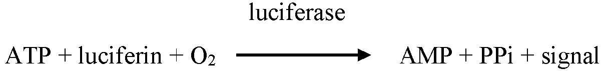

In accordance with the above embodiments, the glyceraldehyde 3-phosphate dehydrogenase, the phosphoglycerate kinase, and/or the adenosine triphosphate sulfurylase may be coupled to a solid support as described above.

The amount of light produced can be easily determined using a sensitive device in the right light such as a luminometer. Thus, the luminometric methods offer the advantage of being capable of quantitation.

In one embodiment, the bioluminescent readout signal is quantified to determine the presence or concentration of the target nucleic acid molecule in the sample. The amount of target nucleic acid can be determined from the peak amplitude of the luminescent signal, and/or the time it takes the signal to reach its peak amplitude, and/or the integrated amount of signal emitted over a period of time, and/or from the rate in which luminescence is produced.

In one embodiment, the target nucleic acid molecule is present in the sample in a concentration of less than 10⁻⁵ moles per liter.

In one embodiment, the presence of the target nucleic acid molecule in the sample is determined by a procedure comprising calculating an initial rate of bioluminescent signal production, calculating what time period is needed to achieve peak bioluminescence, and calculating bioluminescent signal peak amplitude or integrated bioluminescent signal from time zero to peak bioluminescence.

These procedures can be used individually or as part of an analytical method that incorporates two or more procedures for better quantitation. For each procedure or combination of procedures, threshold values can be pre-determined and calibrated to known amounts of target nucleic acid targets. Predetermined values can also be useful in identifying similar but non-identical sequences (i.e., mutations) to the desired target oligonucleotides. Additionally, the values provided by these analytical procedures can be evaluated against a cut-off value to provide a present/absent measurement, or as a scale to enable quantitative readout.

In one embodiment, multiple capture oligonucleotide molecules are provided for detecting multiple target nucleic acid molecules.

The contemplated method in accordance with this embodiment is
a multiplex assay in which a plurality of capture oligonucleotides is utilized to determine whether one or more of a plurality of predetermined nucleic acid target sequences is present or absent in a sample. A particularly useful area for such multiplex assays is in screening assays where the usual analytical output indicates that the sought-after nucleic acid is absent.

In a multiplexed embodiment of the above method, the sample is admixed with a plurality of different capture oligonucleotides. In this embodiment, the analytical output for a certain result with one of the capture oligonucleotides is distinguishable from the analytical output from the opposite result with all of the capture oligonucleotides.

In accordance with this embodiment, for example, a solid support may contain multiple capture oligonucleotides specific for multiple target nucleic acids. Each capture oligonucleotide can be localized at defined positions or regions of the solid support, or synthesized on the surface at defined positions or regions of the solid support *in situ.* Such support facilitates parallel analysis of multiple capture oligonucleotide-bound target nucleic acids. Such supports are also appropriate for high throughput screening.

In certain embodiments, the reactions of the present application are performed in solution. The term "in solution" refers to any assay in which the target nucleic acid is detected while in solution or in suspension. For example, a first hybridization to a target nucleic acid can be performed with a first capture oligonucleotide, and a second hybridization to a target nucleic acid can be performed with a second capture oligonucleotide. Such multiple hybridizations can include a washing step to remove any undesirable (e.g., non-hybridizing sequences) components.

In certain embodiments, the enzymes of the method according to the present application may be coupled to a solid support. In other embodiments, the enzymes of the method may remain in solution.

Suitable supports include organic or inorganic materials and may be of any suitable size or shape (e.g., scaffolds sheets, platforms, and/or nanoparticles). Tethering or immobilizing the components of the assays according to the present application serves to, for example, confine them spatially as well as to enhance their stability and/or function in carrying out, for example, a cascading or sequential reaction as part of the particular assay. In certain embodiments, the support materials include, e.g., nucleotide sequences or gels. In certain embodiments, the enzymes or components of the assays according to the present application may be immobilized on or tethered to, for example, a nanoparticle or the luminal surface of a channel (e.g., a microfluidic channel) of a support material such as a platform.

Several techniques can be used to immobilize components of an assay according to the present application (e.g., enzymes) on surfaces. For example, components may be attached non-specifically or be bound through specific, though non-oriented, chemical reactions (such as carboxy-amide binding). Oriented enzyme immobilization may also be used in accordance with methods of the present application. Oriented enzyme immobilization confers several advantages including, for example, positioning a binding tag (e.g., an affinity tag) so that the activity and stability of the tethered enzyme is optimized (*see* Mukai et al., "Sequential Reactions of Surface-Tethered Glycolytic Enzymes," Chem. Biol. 16(9):1013-20 (2009)).

One example of how an enzyme involved in nucleic acid detection would be tethered to a surface is the use of oriented immobilization. In certain embodiments of the assays according to the present application, recombinant enzymes or assay components which are involved in the assay's reactions are engineered with an affinity tag, enabling them to bind to a surface such as silica or nickel, or a component of a surface such as nickel-nitrilotriacetic acid. For example, an affinity tag could be attached at the amino or carboxy terminus of a protein to be immobilized, or be embedded within the protein to be immobilized. The optimal location of the tethering domain will depend upon the nature and location of the enzyme's catalytic domain(s), substrate binding domain(s), and any conformational changes the enzyme must make.

The use of affinity tagged proteins is especially convenient as the proteins (*i.e.,* DNA polymerase, luciferase, etc.) used in the methods of the present application can readily be expressed as fusions with a suitable binding tag to facilitate immobilization to solid support containing the corresponding capture binding moiety. Suitable capture moieties and binding tag partners that can be used in accordance with this embodiment of the present application include, without limitation, His-Si, His, Si, biotin, streptavidin, Pt, Au, Ag, His-Pt, His-Au, His-Ag, GST, antibody, and epitope tag. Methods of covalently attaching oligonucleotides to a solid support are well known in the art, *see e.g.,* Gosh et al., "Covalent Attachment of Oligonucleotides to Solid Supports," Nucleic Acids Res. 15(13): 5353-5372 (1987), Joos et al., "Covalent Attachment of Hybridizable Oligonucleotides to Glass Supports," Anal. Biochem. 247(1):96-101 (1997); Lund et al., "Assessment of Methods for Covalent Binding of Nucleic Acids to Magnetic Beads, Dynabeads, and the Characteristics of the Bound Nucleic Acids in Hybridization Reactions," Nucleic Acids Res. 16(22):10861-80 (1988).

In certain embodiments, the DNA polymerase and/or the luciferase is coupled to a solid support.

In accordance with this aspect of the present application, the DNA polymerase and/or the luciferase may be coupled to the solid support with a linker selected from the group consisting of His-Si, His, Si, biotin, streptavidin, Pt, Au, Ag, His-Pt, His-Au, His-Ag, GST, antibody, and epitope tag.

The surfaces acting as a support, platform, or scaffold can take multiple forms, including, for example, various nanoparticles, or strands of nucleic acids, and may include various geometries.

In certain embodiments according to the present application, the support is a nanoparticle. As used herein, the term "nanoparticle" refers to any particle the average diameter of which is in the nanometer range, i.e., having an average diameter up to 1 µm. The nanoparticle used can be made of any suitable organic or inorganic matter that will be known to those of ordinary skill in the art. For example, nanoparticles may be composed of any polymer, iron (II,III) oxide, gold, silver, carbon, silica, CdSe and/or CdS. In one embodiment, the nanoparticle is a magnetic nanoparticle. In another embodiment, the nanoparticle is a magnetic, silica-coated nanoparticle ("MSP").

In addition to nanoparticles (NP), supports or scaffolds of different materials can be in the form of rods, planar surfaces, graphene sheets, nanotubes, DNA scaffolds, gels, microspheres, or inner channel walls of a microchannel of a larger support. Quantum dots are also contemplated for use as a support in accordance with the present application. Enzyme immobilization can be attained via non-specific binding, chemical modifications, affinity tags, or other conjugation techniques.

In one embodiment according to the present application, the methods further comprise carrying out a positive and/or negative control. Detection of a diagnostic or prognostic amount of a target nucleic acid is carried out by comparison with a control amount. A control amount of a target nucleic acid can be any amount or a range of amount which is to be compared against a test amount of a target nucleic acid. A control amount may be the amount of a target nucleic acid in a positive or negative control sample carried out as part of the assay according to the present application. A control amount can be either an absolute amount (e.g., µg/ml) or a relative amount (e.g., relative intensity of signals).

Exemplary negative controls for use in the methods of the present application include blocking oligonucleotides targeting the target-oligonucleotide (completely or partially complimentary sequence to the target oligonucleotide), blocking oligonucleotides targeting the capture oligonucleotide (modified at the 3'/5' end to inhibit extension), ribonuclease (RNAse) added to the reaction mixture, a reaction mixture lacking the capture oligonucleotide, a reaction mixture lacking nucleotides (dNTPs), and a reaction mixture lacking any of the substrates. Exemplary positive controls for use in the methods of the present application include various concentrations (including saturating amounts) of target-oligonucleotide mimic (DNA oligonucleotide with an identical sequence of the target-oligonucleotide coming from the sample), and pre-annealed double stranded DNA with overhanging single stranded sequence enabling extension by the polymerase.

In various related aspects, the present application also relates to devices and kits for performing the methods described herein. Such kits contain monitors, reagents and procedures that can be utilized in a clinical or research setting or adapted for either the field laboratory or on-site use. In particular, kits comprising the disclosed reagents used in practicing the methods described herein include any of a number of means for detecting the captured target nucleic acid molecule and measuring the bioluminescent signal produced subsequent to target capture, along with appropriate instructions, are contemplated Suitable kits comprise reagents sufficient for performing an assay to detect a target nucleic acid molecule.

It is to be understood that such a kit is useful for any of the methods of the present application. The choice of particular components is dependent upon the particular method the kit is designed to carry out. Additional components can be provided for detection of the analytical output, as measured by the release of ATP and detection of the bioluminescent signal.

As described above, the kit optionally further comprises instructions for detecting the target nucleic acid nucleic acid by the methods described herein. The instructions present in such a kit instruct the user on how to use the components of the kit to perform the various methods of the present application. These instructions can include a description of the detection methods of the present application, including detection by luminescence.

Accordingly, another aspect of the present application relates to a kit for detecting a target nucleic acid molecule in a sample. The kit includes a capture oligonucleotide molecule complementary to at least a portion of the target nucleic acid molecule so that the capture oligonucleotide molecule hybridizes to a complementary portion of the target nucleic acid molecule and forms a double-stranded nucleic acid molecule, wherein said capture oligonucleotide molecule has (i) a length of 30-60 bases, (ii) a 3' tail consisting of 4 to 8 nucleotides, (iii) a 5' tail, (iv) a target-specific portion between the 3' tail and the 5' tail, (v) a deoxy-adenosine phosphate content of 40-50%, (vi) no deoxy thymidine phosphate in the 3' tail or the 5' tail, and (vii) the 3' tail and the 5' tail collectively comprising a deoxy-adenosine phosphate content which is 40-50% of that of said length of 30-60 bases of the capture oligonucleotide molecule, a polymerase coupled to a solid support; a dNTP mixture, wherein deoxy-adenosine triphosphate is excluded from the dNTP mixture, an enzyme for producing adenosine triphosphates from released free phosphates coupled to a solid support, and a luciferase for producing a bioluminescent readout signal, where the luciferase is coupled to a solid support.

Another aspect of the present application relates to a kit for detecting a target nucleic acid molecule in a sample. The kit includes a capture oligonucleotide molecule complementary to at least a portion of the target nucleic acid molecule so that the capture oligonucleotide molecule hybridizes to a complementary portion of the target nucleic acid molecule and forms a double-stranded nucleic acid molecule. The capture oligonucleotide molecule has (i) a length of 30-60 base pairs, (ii) a 4-8 base pair overhang on its 3' end, (iii) a 5' tail, (iv) a target-specific portion between the 3' end and the 5' tail, (v) a deoxy-adenosine diphosphate content of 40-50%, (vi) no deoxy thymidine phosphate in the 3' end or the 5' tail, and (vii) the 3' end and the 5' tail having an ATP content which is 40-50% of that of the capture oligonucleotide molecule. The kit also includes a polymerase, a dNTP mixture, wherein deoxy-adenosine triphosphate is excluded from the dNTP mixture, an enzyme for producing adenosine triphosphates from released free phosphates, and a luciferase for producing a bioluminescent readout signal.

The kits described above may also comprise multiple capture oligonucleotide molecules for detecting multiple target nucleic acid molecules. In a contemplated kit for multiplexed capture oligonucleotide-mediated specific nucleic acid detection, the kit contains a plurality of capture oligonucleotides for nucleic acid targets of interest. Preferably, where the kits contain multiple capture oligonucleotides, each of the capture oligonucleotides is designed to interrogate a different target nucleic acid sequence.

A final aspect of the present application relates to the use of a composition in the methods of the invention, that comprises a capture oligonucleotide molecule, wherein the capture oligonucleotide molecule has (i) a length of 30-60 base pairs, (ii) a 4-8 base pair overhang on its 3' end, (iii) a 5' tail, (iv) a target-specific portion between the 3' end and the 5' tail, (v) a deoxy-adenosine diphosphate content of 40-50%, (vi) no deoxy thymidine phosphate in the 3' end or the 5' tail, and (vii) the 3' end and the 5' tail having an ATP content which is 40-50% of that of the capture oligonucleotide molecule.

### EXAMPLES

The examples below are intended to exemplify the practice of embodiments of the disclosure but are by no means intended to limit the scope thereof.

### Example 1 - Excluding dATP From the Reaction Mixture Improves Assay Kinetics and Sensitivity

A 100 ul total volume reaction mixture was prepared by mixing 0.05 µl ATP sulfurylase (NEB, M0394S, 300U/ml), 0.25 µl Klenow (NEB, Lg fragment, M0210S, 5000U/ml), 5 µl His-Si-Luciferase (lab made), 5 µl luciferin (200mM), 5 µl of 20X Luciferase buffer (50 mM HEPES, 40 mM KCL, 200 mM MgCl₂), 2 µl APS (30mM), 1 µl Capture-Oligonucleotide (T2 (SEQ ID NO:7), 1 µM), 1.8 µl of dNTP mix (33 mM each) and +/- dATP. The reaction mixture was added into individual wells of a white 96 well plate containing 1 µl Target-oligonucleotide (R6 (SEQ ID NO:6), 1 µM). The reaction mixture was then immediately placed into a TECAN plate reader to read the luminescence signal at room temperature for 2000 seconds with 400msec integration time.

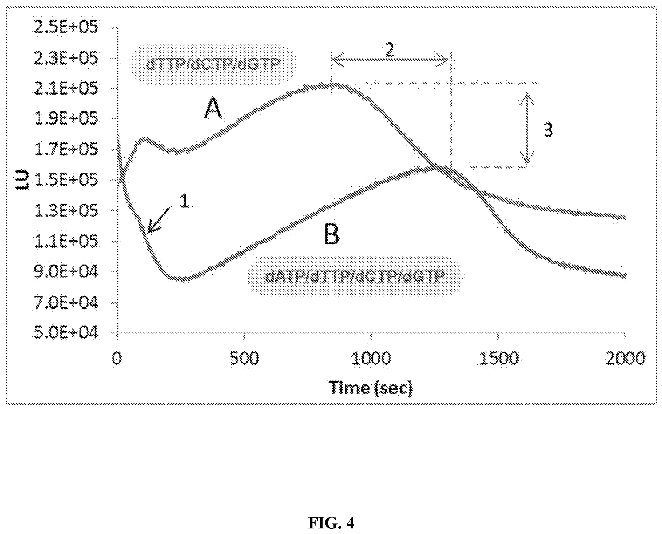

As shown in FIG. 4, two identical reactions were tested using conditions as described above, with the exclusion of dATP (deoxyadenosine triphosphate) from the nucleotide mix added to reaction A. dATP binding to, and hydrolysis, by luciferase causes a decay in the initial luminescence signal (1), delayed peak response phase (2), and overall lower signal amplitude (3). This shows the relationship between pure sequencing and the tethered detection method used here, in which bioluminescence is produced as the read-out. As a result of these data, the capture-oligonucleotide is designed to not include any dTPS in its sequence, to avoid the need for dATP in the reaction mixture.

### Example 2 - The TET-miRNA Assay Can Detect Mismatching Nucleotides in the 3' End of the Target-Oligonucleotide

A 100 µl total volume reaction was prepared by mixing 0.05µl ATP sulfurylase (NEB, M0394S, 300U/ml), 0.25 µl Klenow (NEB, Lg fragment, M0210S, 5000U/ml), 5 µl His-Si-Luciferase (lab made), 5 µl luciferin (200mM), 5 µl of 20X Luciferase buffer (50 mM HEPES, 40 mM KCL, 200 mM MgCl₂), 2 µl APS (30mM), 1 µl Capture-Oligonucleotide (T2 (SEQ ID NO:7), 100 uM), and 1.8 µl of dNTP mix (33mM each). The reaction mixture was added into individual wells of a white 96 well plate containing 1µl of each Target-oligonucleotide (R1-R6 (SEQ ID NOs: 1-6), 100 µM), and immediately placed into a TECAN plate reader to read the luminescence signal at room temperature for 3500 seconds with 400msec integration time.

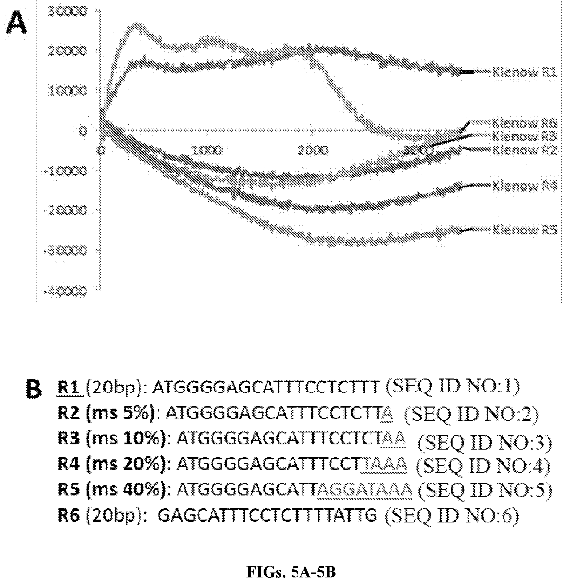

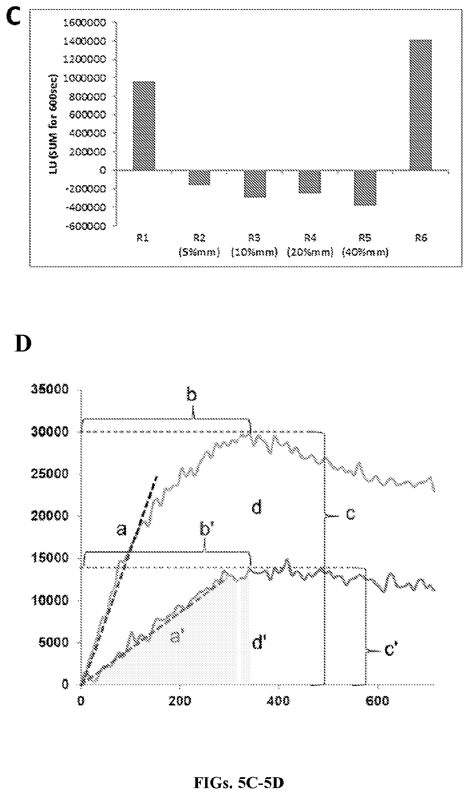

FIG. 5A shows luminescence signal decreases with increasing percent of mismatched nucleotides as indicated in FIG. 5B (indicated in underlined fonts) at the 3' end of the target oligonucleotide sequence. Two complementary target-oligonucleotides (R1 (SEQ ID NO:1) and R6 (SEQ ID NO:6), complementary to shifted and overlapping sequences within the Capture-Oligonucleotide) and 4 mismatched oligonucleotides (presented with the percentage of mismatching nucleotides) were tested, demonstrating strong inhibition of the TET reaction upon nucleotide mismatch and summarized in FIG. 5C. FIG. 5D shows an illustration of possible data analysis procedures for determining target-oligonucleitde presence and hybridization to the Capture-Oligonucleotide (provided for data presented in FIG. 5A for target-oligonucleotides R1 (SEQ ID NO: 1) and R6 (SEQ ID NO:6)) : a/a'- calculating the initial rate of luminescent signal production (slope); b/b' - time to peak luminescence; c/c'- peak amplitude of the luminescent signal; and d/d' the integrated luminescent signal from time 0 to peak.

### Example 3 - Determining the Range of Sensitivity for Target-Oligonucleotide Detection Using the TET-miRNA Assay

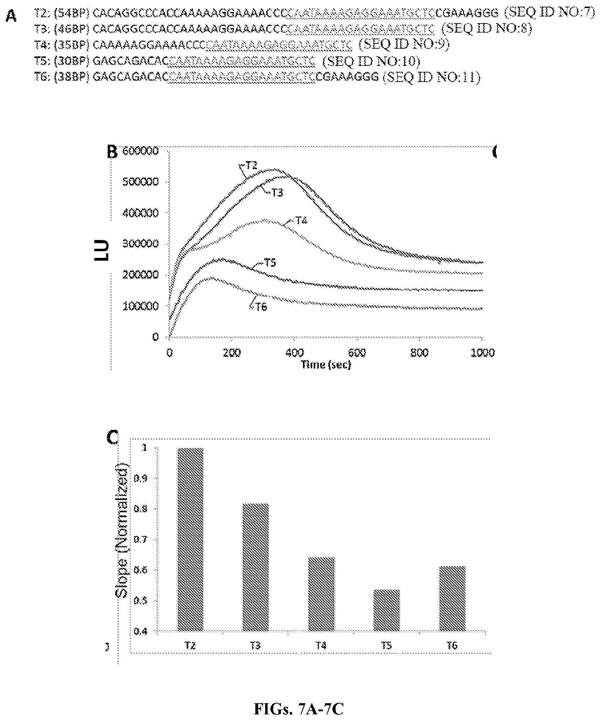

A 100 µl total volume reaction mixture was prepared by mixing 0.05 µl ATP sulfurylase (NEB, M0394S, 300U/ml), 0.25 µl Klenow (NEB, Lg fragment, M0210S, 5000U/ml), 5 µl His-Si-Luciferase (lab made), 5 µl luciferin (200 mM), 5 µl of 20X Luciferase buffer (50 mM HEPES, 40 mM KCL, 200 mM MgCl₂), 2 µl APS (30 mM), 0.5µl Capture-Oligonucleotide (T2 (SEQ ID NO:7)(see FIG. 7A), 1 µM), and 1.8 µl of dCTP/dTTP/dGTP mix (33mM each). The reaction mixture was added into individual wells of a white 96 well plate containing decreasing concentrations of the Target-oligonucleotide (R6 (SEQ ID NO:6), 0mM, 1pM, 10pM, 100pM, 1nM, 10nM, 1µM), and immediately placed into a TECAN plate reader to read the luminescence signal at room temperature for 2000 seconds with 400msec integration time.

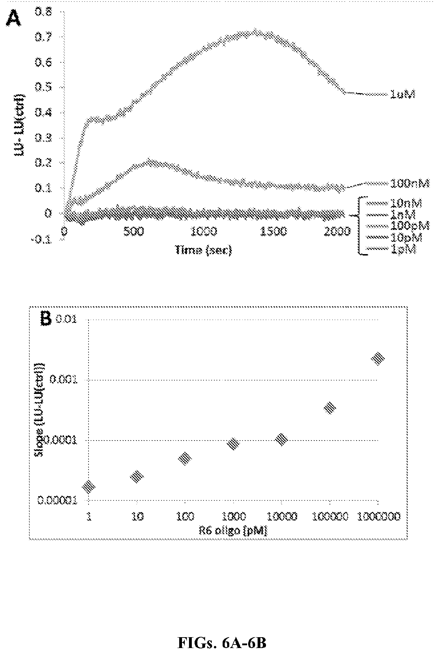

FIG. 6A shows the luminescence signal in response to decreasing concentrations of the target oligonucleotide. A detection range between 1 pico-Molar (10⁻¹² mol/L) to 1 microMolar (10⁻⁶ mol/L) concentrations is demonstrated. FIG. 6B shows that, although further optimization is needed, the summary of FIG. 6A as calculated from the reaction kinetics (i.e., slopes of the initial reaction phase as a function of the oligonucleotide's concentration given in picoM) indicates high sensitivity and a wide dynamic range.

### Example 4 - The Design of 5' and 3' Tails of the Capture-Oligonucleotide Affects TET-miRNA Reaction Kinetics

A100 µl total volume reaction mixture was prepared by mixing 0.05 µl ATP sulfurylase (NEB, M0394S, 300U/ml), 0.25 µl Klenow (NEB, Lg fragment, M0210S, 5000U/ml), 5 ul His-Si-Luciferase (lab made), 5 µl luciferin (200mM), 5 µl of 20X Luciferase buffer (50 mM HEPES, 40 mM KCL, 200 mM MgCl₂), 2 µl APS (30mM), 1µl Target-oligonucleotide (R6 (SEQ ID NO:6), 100 µM), and 1.8 µl of dNTP mix (33mM each). The reaction mixture was added into individual wells of a white 96 well plate containing 1µl of each Capture-Oligonucleotide (T2-T6 (SEQ ID NOs:7-11), 100 µM), and immediately placed into a TECAN plate reader to read the luminescence signal at room temperature for 1000 seconds with 400msec integration time.

As shown in FIG. 7A, several designs of capture oligonucleotides were generated (T2-T6 (SEQ ID NOs:7-11)). All designs include a similar complementary insert sequence (complementary to the R6 target-oligonucleotide, indicated by the underlined font). FIG. 7B shows the luminescence signal as measured in the presence of the various capture oligonucleotides (as provided in FIG. 7A), in response to addition of the target-oligonucleotide. FIG. 7C is a summary of FIG. 7B and demonstrates the differences in annealing kinetics, as indicated by the calculated reaction slopes for the various capture-oligonucleotides.

### Example 5 - The Capture-Oligonucleotide dATP Content Affects TET-miRNA Reaction Kinetics

A 100 µl total volume reaction mixture was prepared by mixing 0.05 µl ATP sulfurylase (NEB, M0394S, 300U/ml), 0.25 µl Klenow (NEB, Lg fragment, M0210S, 5000U/ml), 5 µl His-Si-Luciferase (lab made), 5 µl luciferin (200 mM), 5 µl of 20X Luciferase buffer (50 mM HEPES, 40 mM KCL, 200 mM MgCl₂), 2 µl APS (30mM), 1 µl Target-oligonucleotide (R6 (SEQ ID NO:6), 100 µM), and 1.8 µl of dNTP mix (33mM each). The reaction mixture was added into individual wells of a white 96 well plate containing 1 µl of each capture-oligonucleotide (T1 (SEQ ID NO:12) + T1A-T1E (SEQ ID NOs:13-17), 100µM), and immediately placed into a TECAN plate reader to read the luminescence signal at room temperature for 1000 seconds with 400msec integration time.

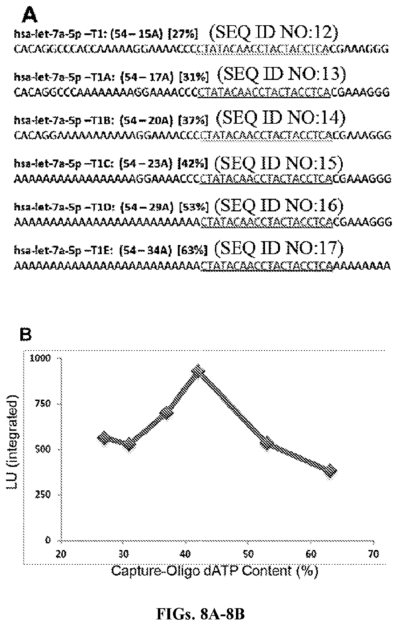

As shown in FIG. 8A, 6 capture-oligonucleotides containing increasing percentages of dATP (27%-63%) were designed for detection of the target-oligonucleotide, hsa-let-7a-5p (all have similar complimentary insert sequence, indicated in underlined fonts). FIG. 8B is a summary of the data that shows optimal activity with 40%-50% dATP content within the capture-oligonucleotide sequence. This finding is surprising and represents a significant enhancement.

### Example 6 - The 3' Tail Length of the Capture-Oligonucleotide Affects TET-miRNA Reaction Activity and Kinetics

A 100µl total volume reaction mixture was prepared by mixing 0.05 µl ATP sulfurylase (NEB, M0394S, 300U/ml), 0.25 µl Klenow (NEB, Lg fragment, M0210S, 5000U/ml), 5 µl His-Si-Luciferase (lab made), 5 µl luciferin (200 mM), 5µl of 20X Luciferase buffer (50 mM HEPES, 40 mM KCL, 200 mM MgCl₂), 2 µl APS (30mM), 1 µl Target-oligonucleotide (R6 (SEQ ID NO:6), 100 µM) and 1.8 µl of dNTP mix (33 mM each). The reaction mixture was added into individual wells of a white 96 well plate containing 1 µl of each cap-oligonucleotide (T1-T9 (SEQ ID NO:12 and SEQ ID NOs:18-25), 100 µM), and immediately placed into a TECAN plate reader to read the luminescence signal at room temperature for 1000 seconds with 400 msec integration time.

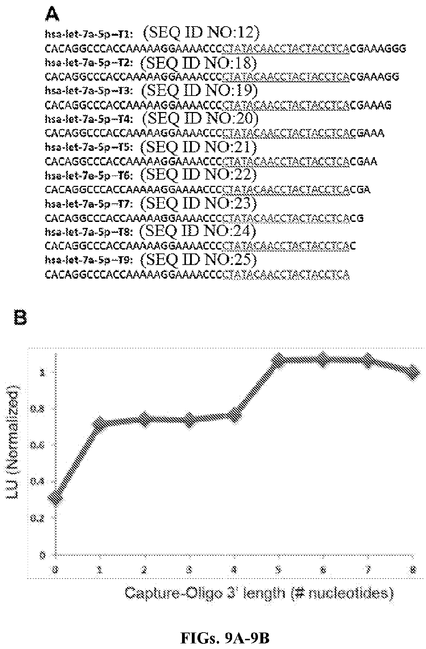

As shown in FIG. 9A, nine capture-oligonucleotides were designed for detection of the target-oligonucleotide, hsa-let-7a-5p. The nine capture-oligonucleotides contain a decreasing number of nucleotides at the 3' end of their sequence following the miRNA complementary insert site (indicated in underlined font). FIG. 9B shows that the TET-miRNA assay optimal activity is reached when 5-8 nucleotides are added on to the 3' of the target-oligonucleotide complementary insert sequence.

### Example 7 - The TET-miRNA Assay is Sensitive to Mutations in the Sequence of MIR-340 Target-Oligonucleotide

A 100 µl total volume reaction mixture was prepared by mixing 0.05 µl ATP sulfurylase (NEB, M0394S, 300U/ml), 0.25 µl Klenow (NEB, Lg fragment, M0210S, 5000U/ml), 5 µl His-Si-Luciferase (lab made), 5 µl luciferin (200mM), 5µl of 20X Luciferase buffer (50 mM HEPES, 40 mM KCL, 200 mM MgCl₂), 2 µl APS (30 mM), 1 µl capture-oligonucleotide (T2, 100 µM), and 1.8 µl of dNTP mix (33 mM each). The reaction mixture was added into individual wells of a white 96 well plate containing 1 µl of each target-oligonucleotide (MIR-340#1-MIR-340#7 (SEQ ID NOs:27-33), 100 µM), and immediately placed into a TECAN plate reader to read the luminescence signal at room temperature for 1200 seconds with 400 msec integration time.

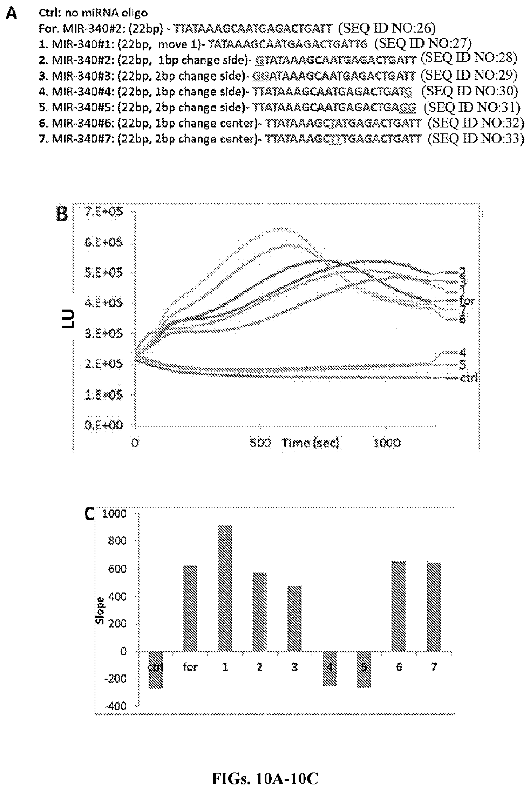

As shown in FIG. 10A, MIR340 target-oligo (MIR-340#1 (SEQ ID NO:27)) and various mutated sequences were used in this experiment (MIR-340#2-#7) (SEQ ID NOs:28-33), and 2 controls (FOR-unmutated MIR-340#2, and Ctrl- a reaction mixture excluding any target-oligonucleotide), mutated nucleotides indicated in underlined fonts). FIGs. 10B-10C show the luminescence signal and kinetics as measured for various mutations as indicated in FIG. 10A. The data in FIG. 10A shows that all of the tested mutations induced detectable differences in the measured signal.

### Example 8 - The TET-miRNA Assay can Detect Naturally Occurring miRNA in Human Serum and Human Plasma

A 40µl total volume reaction mixture was prepared by mixing 0.05 µl ATP sulfurylase (NEB, M0394S, 300U/ml), 0.25 µl Klenow (NEB, Lg fragment, M0210S, 5000U/ml), 5 µl His-Si-Luciferase (lab made), 5 µl luciferin (200mM), 5µl of 20X Luciferase buffer (50 mM HEPES, 40 mM KCL, 200 mM MgCl₂), 2 µl APS (30 mM), 1 µl capture-oligonucleotide targeting naturally occurring miRNA as listed in FIG. 10A (100 µM), and 1.8 µl of dNTP mix (33 mM each). The reaction mixture was added into individual wells of a white 96 well plate containing 60 µl of human serum or human plasma, and immediately placed into a TECAN plate reader to read the luminescence signal at room temperature for 500 seconds with 400 msec integration time.

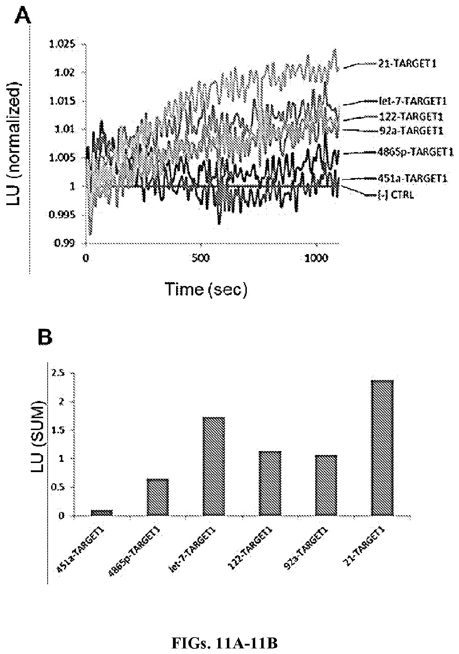

As shown in FIG. 11A, six capture-oligonucleotides were designed to detect 6 different naturally occurring miRNAs in commercially obtained human serum. The real-time kinetics of the TET-miRNA reaction are shown. FIG. 11B is a summary of FIG. 11A, where the bioluminescence signal was integrated for 500 seconds to show the relative amounts of the various miRNAs in the tested serum sample.

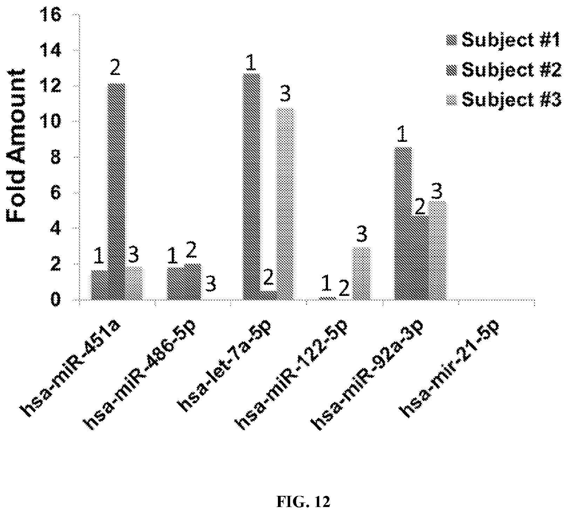

As shown in Fig. 12, plasma samples from 3 human donors (collected at the Guthrie Medical Center, Sayre PA) were tested with TET-miRNA for a panel of 6 naturally occurring miRNAs.

### Example 9 - Ribonuclease (RNAse) Treatment Abolishes the TET-miRNAs Activity

A 40 µl total volume reaction mixture was prepared by mixing 0.05 µl ATP sulfurylase (NEB, M0394S, 300U/ml), 0.25 µl Klenow (NEB, Lg fragment, M0210S, 5000U/ml), 5 µl His-Si-Luciferase (lab made), 5 µl luciferin (200 mM), 5 µl of 20X Luciferase buffer (50 mM HEPES, 40 mM KCL, 200 mM MgCl₂), 2µl APS (30 mM), 1 µl capture-oligonucleotide targeting naturally occurring miRNA as described in FIGs. 12A-12B (100 µM), and 1.8 µl of dNTP mix (33 mM each). The reaction mixture was added into individual wells of a white 96 well plate containing 60 µl of human plasma, and immediately placed into a TECAN plate reader to read the luminescence signal at room temperature for 500 seconds with 400msec integration time.

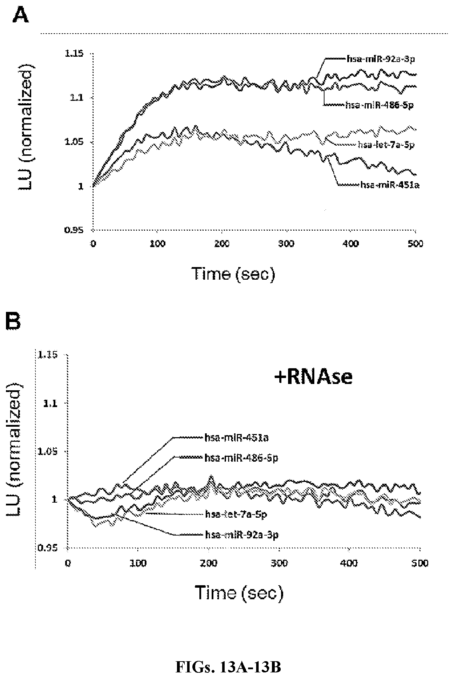

FIG. 13A shows plasma samples from 3 subjects (collected from Guthrie (Sayre, PA)) that were tested with TET-miRNA for a panel of 4 naturally occurring miRNAs. FIG. 13B shows a significant reduction in signal observed following treatment with RNAse-A for 30 minutes at room temperature.

### Example 10 - Analysis of Target-Oligonucleotide Detection with NP-Immobilized vs. In-Solution Reactions

Biotinylated enzymes were immobilized onto streptavidin coated microspheres (500 nm SiO₂, Bangs Laboratory, IN, USA) according to manufacturer instructions, and then spin washed of unbound protein 3 times. Equal amounts of NP tethered or untethered enzymes were added to a reaction mixture containing 100 µl total volume per reaction of: 5µl His-Si-Luciferase (lab made), 5 µl luciferin (200mM), 5 µl of 20X Luciferase buffer (50 mM HEPES, 40 mM KCL, 200 mM MgCl₂), 2 µl APS (30 mM), 1 µl Capture-Oligonucleotide (Cap-HAS-MIR-451a, 1 µM), and 1.8 µl of dCTP/dTTP/dGTP mix (33 mM each). The reaction mixture was added into individual wells of a white 96 well plate containing the target-oligonucleotide (HAS-MIR-451a, 1 µM), and immediately placed into a TECAN plate reader to read the luminescence signal at room temperature for 2000 seconds with 400 msec integration time.

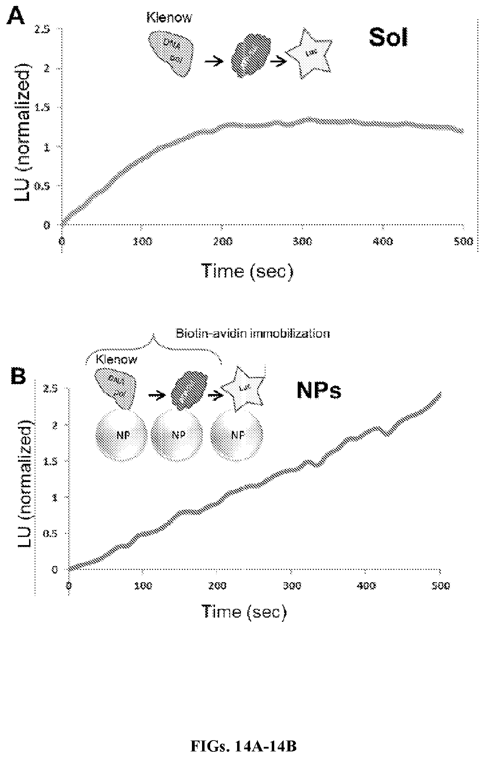

Commercially obtained human serum samples were spiked with equal amounts of the target-oligonucleotide, HSA-MIR-451a, and added to TET-miRNA reaction mixtures including soluble enzymes Luciferase/ATP-Sulfurylase/Klenow (FIG. 14A, Sol), or tethered to NPs (FIG. 14B, NP). Note that non-oriented immobilization via biotinylation was used to tether the DNA-polymerase and ATP-sulfurylase to NPs. ATP sulfurylase (NEB, M0394S, 300U/ml) and Klenow (NEB, Lg fragment, M0210S, 5000U/ml) were biotinylated using the EZ-Link Sulfo-NHS-LC-Biotinylation Kit (Thermo Scientific, USA) according to manufacturer instructions.

### Example 11 - Immobilization of Commercial BST2.0 onto NPs Via Biotin-Streptavidin Binding Inhibits the TET-miRNA Reaction Assay

BST2.0 (NEB, M0537S, 8000U/ml) was biotinylated using the EZ-Link Sulfo-NHS-LC-Biotinylation Kit (Thermo Scientific, USA) according to manufacturer instructions. The biotinylated enzyme was immobilized onto streptavidin coated microspheres (500 nm SiO₂, Bangs Laboratory, IN, USA) according to manufacturer instructions, and then spin washed of unbound protein 3 times. Equal amounts of NP tethered or untethered BST2.0 was added to a reaction mixture containing 100 µl total volume per reaction of: 0.05 µl ATP sulfurylase (NEB, M0394S, 300U/ml), 5 µl His-Si-Luciferase (lab made), 5 µl luciferin (200 mM), 5 µl of 20X Luciferase buffer (50 mM HEPES, 40 mM KCL, 200mM MgCl₂), 2 µl APS (30mM), 1 µl Capture-Oligonucleotide (Cap-HSA-MIR-451a, 1 µM), and 1.8 µl of dCTP/dTTP/dGTP mix (33 mM each). The reaction mixture was added into individual wells of a white 96 well plate containing the target-oligonucleotide (HSA-MIR-451a, 1 µM), and immediately placed into a TECAN plate reader to read the luminescence signal at room temperature for 2000 seconds with 400 msec integration time.

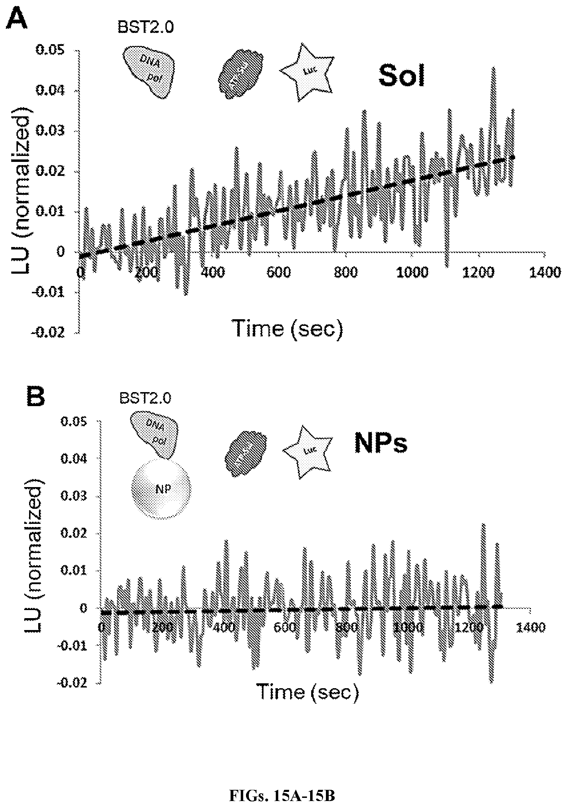

Commercially obtained human serum samples were spiked with equal amounts of HSA-MIR-451a and added to TET-miRNA reaction mixtures including soluble enzymes Luciferase/ATP-Sulfurylase/Bst2.0 (FIG. 15A, Sol), or when Bst2.0 was immobilized onto NPs via biotinylation (FIG. 15B, NP). The data show that biotinylation had a negative effect on its activity, and even more so when Bst2.0 was tethered to NPs via non-oriented immobilization.

### Example 12 - TET-miRNA Assay is Only Marginally Affected by Temperature

A 100 µl total volume reaction mixture was prepared by mixing 0.05 µl ATP sulfurylase (NEB, M0394S, 300U/ml), 0.25 µl Klenow (NEB, Lg fragment, M0210S, 5000U/ml), 5 µl His-Si-Luciferase (lab made), 5 µl luciferin (200 mM), 5 µl of 20X Luciferase buffer (50 mM HEPES, 40 mM KCL, 200 mM MgCl₂), 2µl APS (30 mM), 1 µl Capture-Oligonucleotide targeting the Has-let-7a-5p miRNA (100 µM), 1.8 µl of dNTP mix (33 mM each), and Has-let-7a-5p oligo. The reaction mixture was added into individual wells of a white 96 well plate, and immediately placed into a TECAN plate reader to read the luminescence signal at various temperatures as indicated. Luminescence was measured for 1000 seconds with 400 msec integration time.

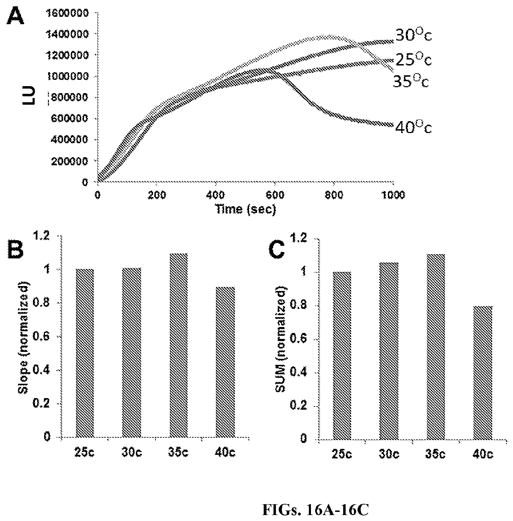

As shown in FIG. 16A, commercially obtained human serum samples were spiked with equal amounts of target-oligonucleotides and added to TET-miRNA reaction mixtures at varying temperatures (25°C-40°C). Only minor differences were observed in the initial parameters of the reaction's kinetics (as measured from the slope, FIG. 16B) and efficiency (as measured from the integrated signal, FIG. 16C).

### Example 13 - Analysis of Two Different Capture-Oligonucleotide Designs for Detecting miRNA in Human Serum

A 100 µl total volume reaction mixture was prepared by mixing 0.05 µl ATP sulfurylase (NEB, M0394S, 300U/ml), 0.25 µl Klenow (NEB, Lg fragment, M0210S, 5000U/ml), 5 µl His-Si-Luciferase (lab made), 5 µl luciferin (200mM), 5 µl of 20X Luciferase buffer (50 mM HEPES, 40 mM KCL, 200 mM MgCl₂), 2 µl APS (30 mM), 1 µl of either CAP1 (SEQ ID NO:34) or CAP2 (SEQ ID NO:35) capture-oligonucleotide (100 µM), 1.8 µl of dNTP mix (33 mM each), and test oligonucleotides. The reaction mixture was added into individual wells of a white 96 well plate, and immediately placed into a TECAN plate reader to read the luminescence signal at RT. Luminescence was measured for 1000 seconds with 400 msec integration time.

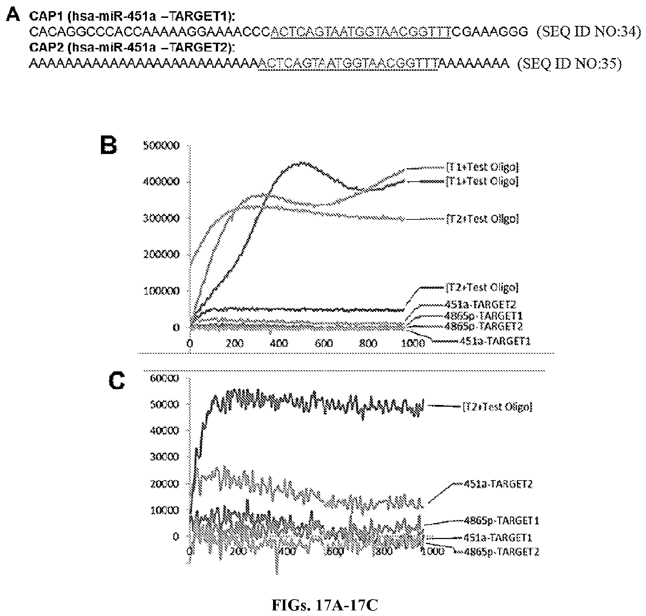

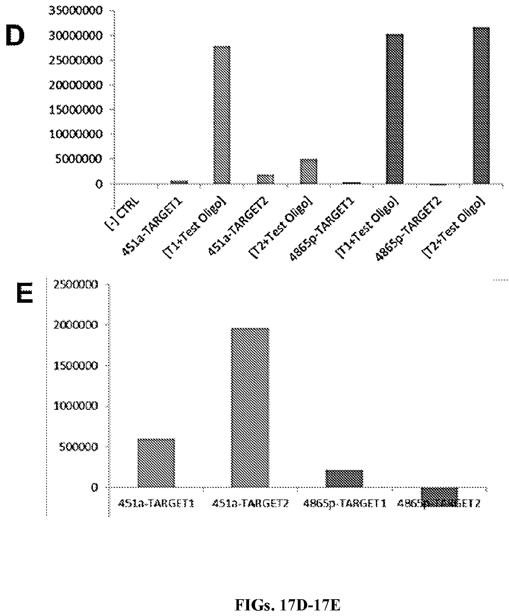

FIG. 17A depicts the design of 2 capture-oligonucleotides that were tested to detect 6 different naturally occurring miRNAs in human plasma. CAP1 is a random nucleotide sequence, and, in CAP2, the 5' tail and 3' tail contain only adenosines. FIG. 17B shows real-time kinetics of the TET-miRNA reaction detecting both naturally occurring miRNAs, as well as DNA based target oligonucleotides that are of similar sequences (test oligos). FIG. 17C is an enlarged portion of the data presented in FIG. 17B to show the luminescent signal of the various miRNA molecules. FIG. 17D is a summary of FIG. 17A, where the luminescence signal was integrated for 500 seconds to show the relative amounts of various miRNAs. FIG. 17E is a summary of measurements of only the naturally occurring miRNAs (as shown in FIG. 17C).

### Example 14 - Comparison of Various DNA Polymerase Activities in the TET-miRNA Assay

A 100 µl total volume reaction mixture was prepared by mixing 0.05 µl ATP sulfurylase (NEB, M0394S, 300U/ml), DNA polymerase (as indicated in FIG. 17A), 5 µl His-Si-Luciferase (lab made), 5 µl luciferin (200 mM), 5 µl of 20X Luciferase buffer (50 mM HEPES, 40 mM KCL, 200 mM MgCl₂), 2µl APS (30 mM), 1 µl Capture-Oligonucleotide targeting hsa-let-7a-5p miRNA (100 µM), 1.8 µl of dNTP mix (33 mM each), and hsa-let-7a-5p oligonucleotide. The reaction mixture was added into individual wells of a white 96 well plate, and immediately placed into a TECAN plate reader to read the luminescence signal at room temperature for 500 seconds with 400 msec integration time.

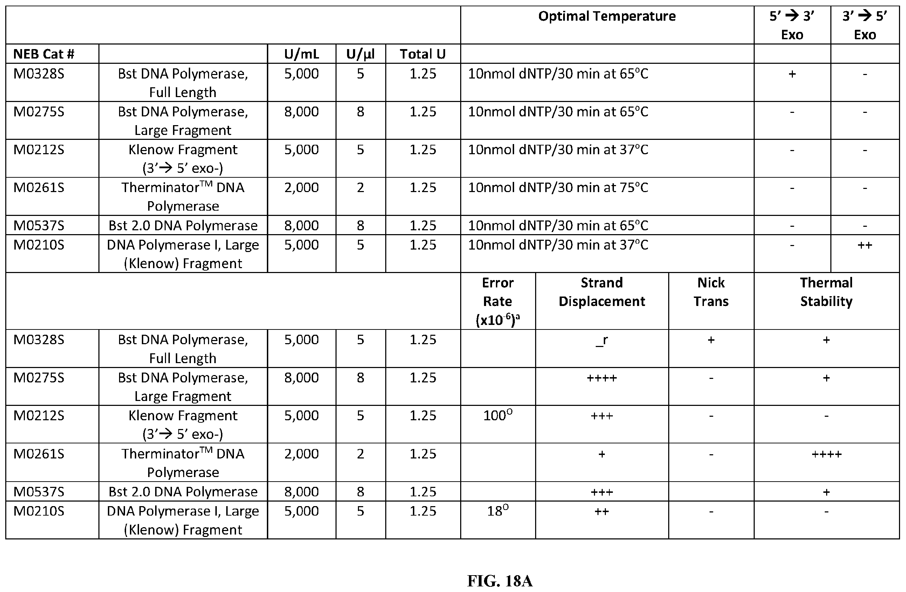

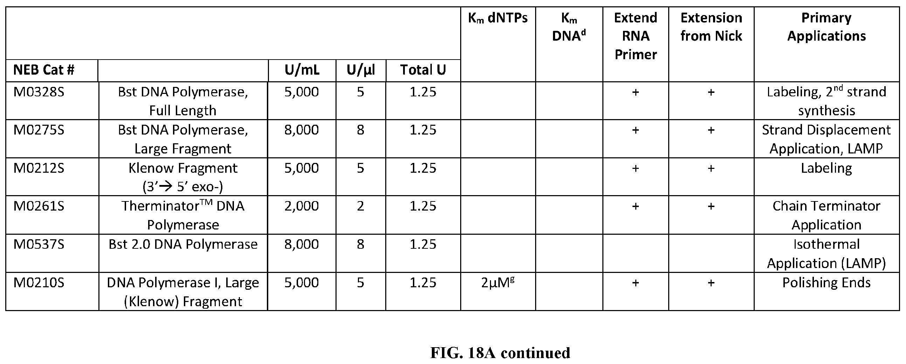

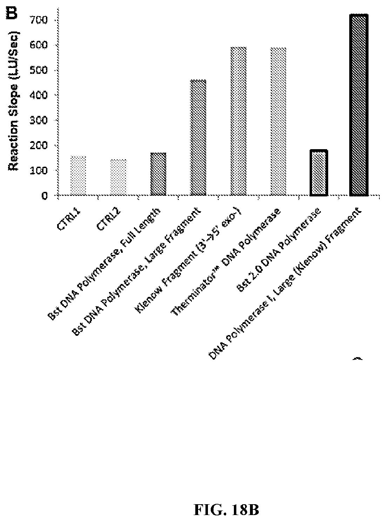

FIG. 18A is a list of the DNA polymerases used in this experiment, and some of their key features. As shown in FIG. 18B, commercially obtained human serum samples were spiked with equal amounts of miRNA oligonucleotides and added to TET-miRNA reaction mixtures (at room-temperature) in the presence of various DNA-Polymerases. The bar plot of the reaction kinetics (calculated from the reaction's initial slope) shows that Bst, Klenow and Terminator DNA-Poly provides the fastest reaction kinetics under these experimental conditions. Control 1 (CTRL1) reaction lacks the Capture-oligonucleotide, while control 2 (CTRL2) does not include a DNA-Polymerase. Data bars for the Bst and Klenow variants used to obtain the data in previous figures are highlighted.

### Example 15 - Target-Oligonucleotide Detection Using TET-miRNA when Tethered Versus In Solution

A reaction mixture consisting of equal amounts of NP tethered or untethered enzymes, 5 µl His-Si-Luciferase, 5 µl luciferin (200 mM), 5 µl of 20X Luciferase buffer (50 mM HEPES, 40mM KCL, 200 mM MgCl₂), 2 µl APS (30 mM), 1 µl Capture-Oligonucleotide (T2 (SEQ ID NO:7), 100 µM), and 1.8 µl of dCTP/dTTP/dGTP mix (33 mM each) was prepared. The reaction mixture was added into individual wells of a white 96 well plate containing increasing amounts of target oligonucleotides (R6 (SEQ ID NO:6), 200 nM, 500 nM, and 1 uM), and immediately placed into a TECAN plate reader to read the luminescence signal at room temperature for 1400 seconds with 400 msec integration time.

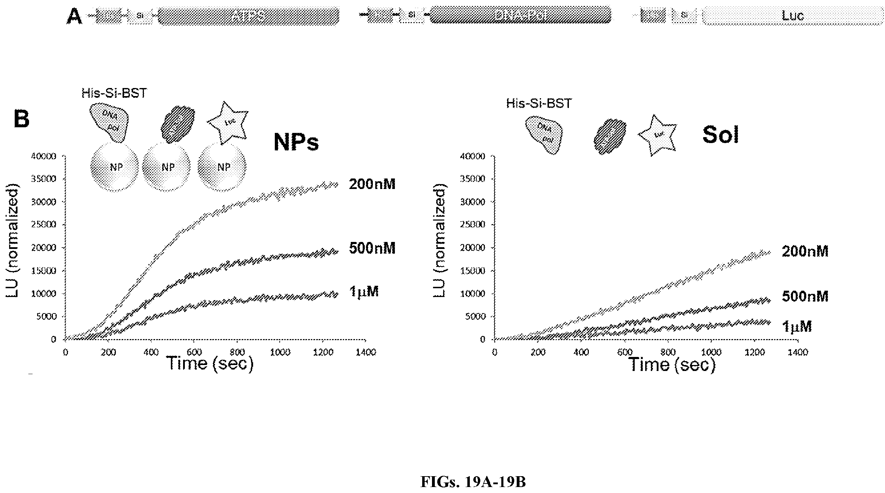

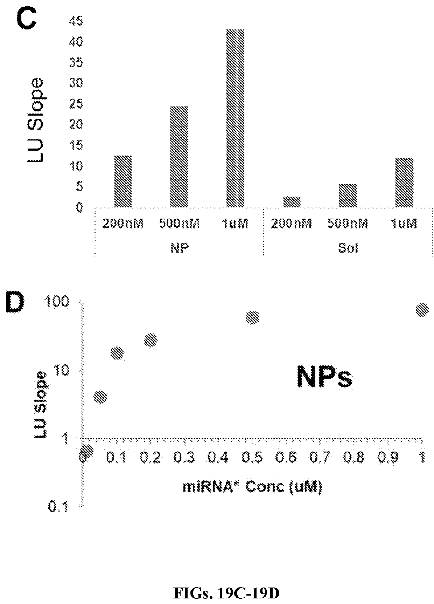

FIG. 19A shows schematic illustrations of the His-Si-enzymes design (ATPS: ATP-sulfurylase, DNA-Pol: DNA polymerase, Luc: luciferase). The genes encoding ATP-sulfurylase (MET3, sulfate adenylyltransferase, *Saccharomyces cerevisiae*), BST (*Bacillus stearothermophilus* DNA polymerase I (pol) gene) and Klenow (*Escherichia coli* strain LD93-1 DNA polymerase I) were inserted in fusion with the His-Si tag into the pET17b vector for bacterial protein expression. The His-Si-proteins were purified using Ni-NTA beads, and stored in native protein buffer (NPB) with sorbitol until use. FIG. 19B shows measurements of enzyme activity when tethered vs. in solution made using the reaction mixture described above. The data shows the kinetics of individual reactions when TET-miRNA enzymes are immobilized onto 500 nm SiO₂ NPs (NPs, left), or when in solution (Sol, right). FIG. 19C is a bar plot of the reaction kinetics (calculated from the reaction's initial slope) that shows that when TET-miRNA enzymes are tethered to NPs the coupled reactions are occurring with faster kinetics. FIG. 19D is a summary of NP TET-miRNA reaction in detection of increasing concentrations of the R6 target oligonucleotide.

### Example 16 - Comparison of His-Si-Klenow Versus His-Si-BST Activity in the TET-miRNA Assay (Tethered versus Non-Tethered)

Measurements of enzyme activity when tethered vs. in solution were made using a reaction buffer consisting of equal amounts of NP tethered or untethered enzymes, 5 µl His-Si-Luciferase, 5 µl luciferin (200 mM), 5 µl of 20X Luciferase buffer (50 mM HEPES, 40 mM KCL, 200 mM MgCl₂), 2 µl APS (30 mM), 1 µl Capture-Oligonucleotide (T2 (SEQ ID NO:7), 100 µM), and 1.8 µl of dCTP/dTTP/dGTP mix (33 mM each). The reaction mixture was added into individual wells of a white 96 well plate containing increasing amounts of target oligonucleotides (R6 (SEQ ID NO:6), 1 µM) and immediately placed into a TECAN plate reader to read the luminescence signal at room temperature for 1200 seconds with 400 msec integration time.

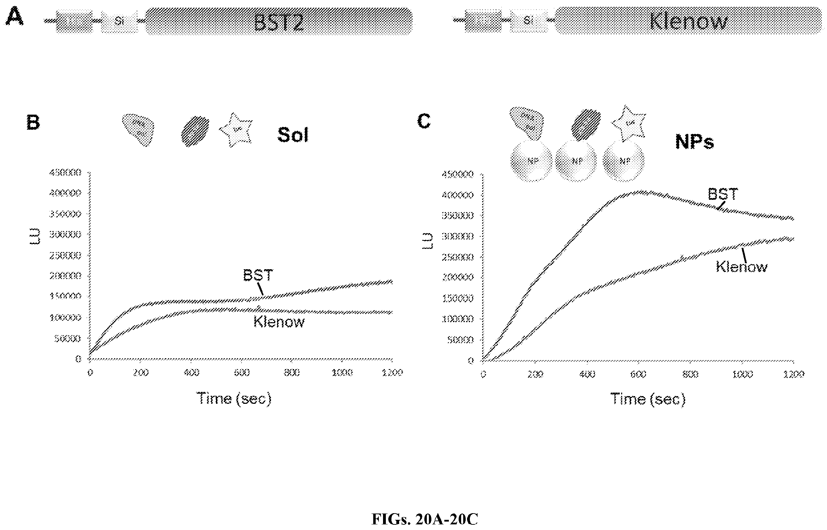

As shown in FIG. 20A, the large Klenow fragment from *E. coli* (*Escherichia coli* strain LD93-1 DNA polymerase I) was expressed as a fusion protein downstream of the His-Si affinity tag. FIG. 20B shows the activity in solution of His-Si-Klenow compared to that of DNA-pol I, His-Si-BST2 (from *Bacillus StearoThermophilus*). His-Si-BST demonstrated slightly better activity kinetics as far as initial reaction rate and overall activity. FIG. 20C shows a comparison of the two DNA-polymerase activities when the reaction enzymes are tethered to 500 nm SiO₂ nanoparticles. Again, His-Si-BST demonstrated better activity. Moreover, both Klenow and BST demonstrated improved activity when the reaction included tethered enzymes.

### Example 17 - Comparison of Target-Oligo Detection Using Various Ratios of His-Si-ATPS/His-Si-BST/NPs

Measurements of enzyme activity were made using a reaction mixture consisting of 20 µl NP-His-Si-BST (at varying ratios of enzyme/NP, as indicated in FIG. 20B), 20 µl NP-His-Si-ATPS, 5 µl His-Si-Luciferase, 5 µl luciferin (200 mM), 5 µl of 20X Luciferase buffer (50 mM HEPES, 40 mM KCL, 200 mM MgCl₂), 2 µl APS (30 mM), 1 µl Capture-Oligonucleotide (T2 (SEQ ID NO:7), 100 µM), and 1.8 µl of dCTP/dTTP/dGTP mix (33 mM each). The reaction mixture was added into individual wells of a white 96 well plate containing 1 µl target oligo (R6, 100 µM) and immediately placed into a TECAN plate reader to read the luminescence signal at room temperature for 2000 seconds with 400 msec integration time.

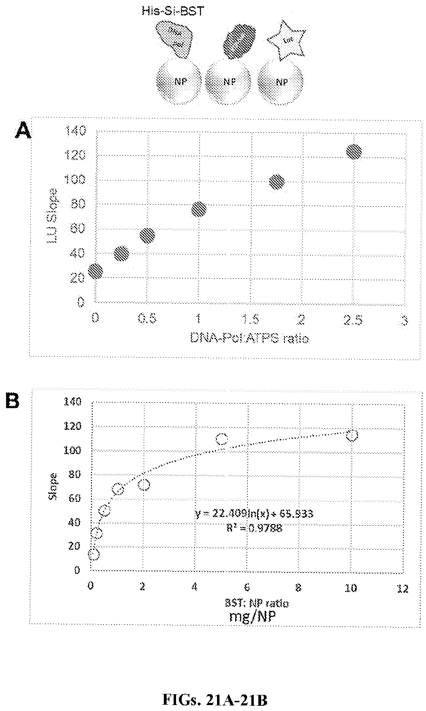

FIG. 21A is a summary of experiments where the ratio of His-Si-BST2 (DNA-Pol) to ATPS is increasing from 0.1:1 to 2.5:1. FIG. 21B is a summary of experiments where the ratio of His-Si-BST2 (DNA-Pol) to NPs is increased from 0.1:1 to 10:1.

### Example 18 - miRNA (RNA Oligo) Detection Using TET-miRNA (His-Si-Enzymes) and DNA Cap-Oligonucleotide

Measurements of enzyme activity were made using a reaction mixture consisting of 20 µl NP-His-Si-BST, 20 µl NP-His-Si-ATPS, 5 µl NP-His-Si-Luciferase, 5 µl luciferin (200 mM), 5 µl of 20X Luciferase buffer (50 mM HEPES, 40 mM KCL, 200 mM MgCl₂), 2 µl APS (30 mM), 1 µl Capture-Oligonucleotide (T2 (SEQ ID NO:7), 100 µM), and 1.8 µl of dCTP/dTTP/dGTP mix (33 mM each). The reaction mixture was added into individual wells of a white 96 well plate containing DNA or RNA target oligonucleotides (R5 (SEQ ID NOs: 38 and 39) or R6 (SEQ ID NOs: 36 and 37), 1 µM) and immediately placed into a TECAN plate reader to read the luminescence signal at room temperature for 2500 seconds with 400 msec integration time.

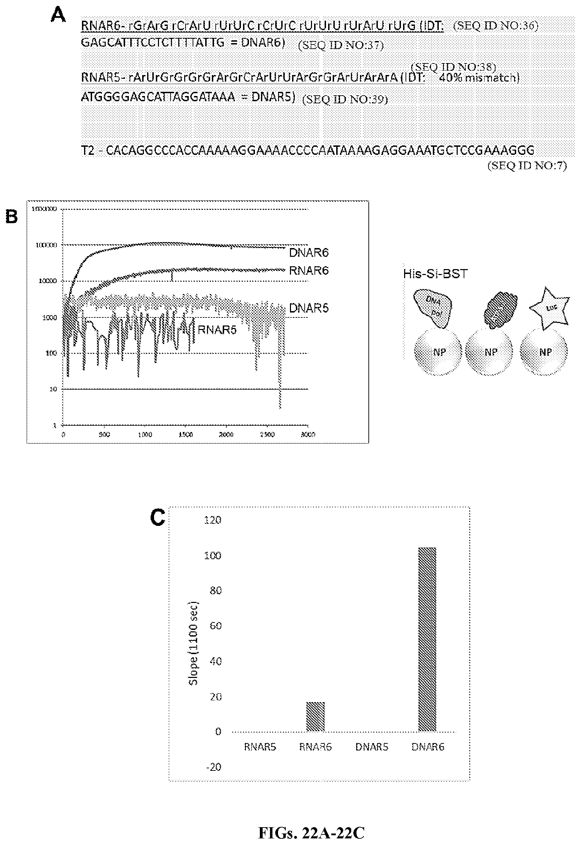

FIG. 22A shows the sequence of the target oligonucleotides (RNA and DNA) as provided by the manufacturing company (IDT, San Diego CA), and the sequence of the CAP-Oligo (T2) designed to detect them. FIG. 22B shows a TET-miRNA reaction using tethered enzymes to detect RNA vs. corresponding DNA target oligonucleotides. FIG. 22C is a summary of data presented in FIG. 22A, showing the calculated initial rates of the reaction (slope).

### Example 19 - Analysis of the Target-Oligo Mismatch Sensitivity of Different Non-Tethered DNA Polymerases (His-Si-Klenow Versus His-Si-BST)

Measurements of enzyme activity were made using a reaction mixture consisting of 20 µl NP-His-Si-BST or NP-His-Si-Klenow, 20 µl NP-His-Si-ATPS, 5 µl NP-His-Si-Luciferase, 5 µl luciferin (200 mM), 5 µl of 20X Luciferase buffer (50 mM HEPES, 40 mM KCL, 200 mM MgCl₂), 2 µl APS (30 mM), 1 µl Capture-Oligonucleotide (T2 (SEQ ID NO:7), 100 µM), and 1.8 µl of dCTP/dTTP/dGTP mix (33 mM each). The reaction mixture was added into individual wells of a white 96 well plate containing 1 µl of each target oligonucleotide (1 µM) and immediately placed into a TECAN plate reader to read the luminescence signal at room temperature for 2000 seconds with 400 msec integration time.

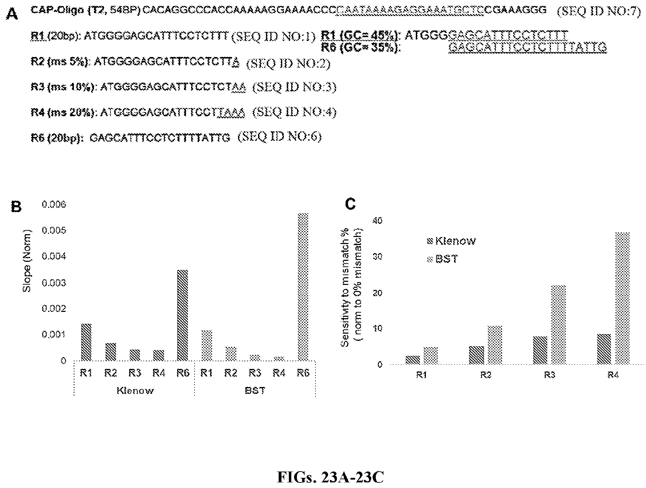

As shown in FIG. 23A, an increasing percent of mismatched nucleotides were introduced into the target oligonucleotides tested here (indicated in underlined font) at the 3' end of the target oligo sequence, against the sequence of the CAP-Oligo T2. Both R1 (SEQ ID NO:1) and R6 (SEQ ID NO:6) oligonucleotides match the CAP-Oligo sequence; however, they contain different levels of GC content. FIG. 23B is a summary of the initial speed of the TET-miRNA reaction to the various oligonucleotides and shows a significantly faster kinetics for the 100% match sequences with both Klenow and BST, as calculated from the initial reaction slope. FIG. 23C demonstrates that calculating the ratio between reaction speeds in the presence of the various mismatch oligonucleotides and the 100% match oligonucleotide provides a measure of how sensitive the two DNA-Polymerases are to mismatch content in the target oligonucleotide, where the BST polymerase is 2-5 times more sensitive depending on the mismatch percentage.

### Example 20 - Tethering the TET-miRNA Reaction when the CAP-Oligo is in Solution Versus Immobilized (Non-Oriented)

Measurements of enzyme activity were made using a reaction mixture consisting of 20 µl NP-His-Si-BST or NP-His-Si-Klenow, 20 µl NP-His-Si-ATPS, 5 µl NP-His-Si-Luciferase, 5 µl luciferin (200 mM), 5 µl of 20X Luciferase buffer (50 mM HEPES, 40 mM KCL, 200 mM MgCl₂), 2 µl APS (30 mM), 1 µl Capture-Oligonucleotide (T2 (SEQ ID NO:7), 100 µM), and 1.8 µl of dCTP/dTTP/dGTP mix (33 mM each). The reaction mixture was added into individual wells of a white 96 well plate containing 1 µl of the target oligonucleotide (1 µM) and immediately placed into a TECAN plate reader to read the luminescence signal at room temperature for 1500 seconds with 400msec integration time.

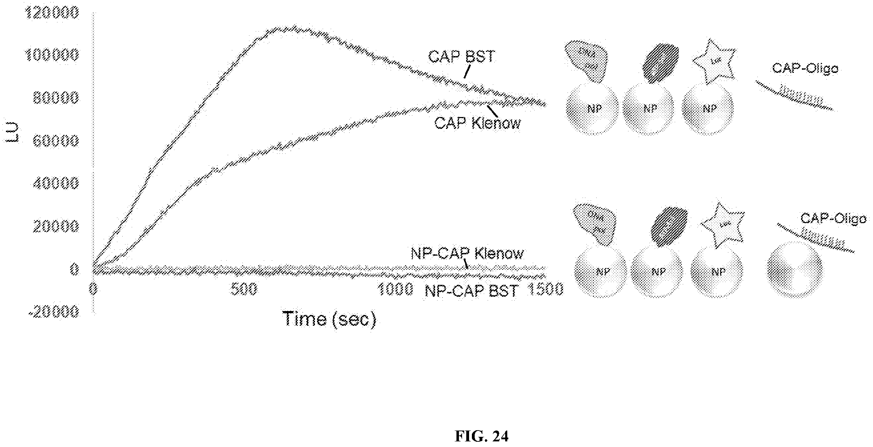

The TET-miRNA reaction was carried out using either a CAP oligonucleotide in solution, or when immobilized onto a SiO₂ NP by non-specific adsorption (Cap-oligo was incubated with SiO₂ NPs at RT for 30 minutes, and then spin washed into natural protein buffer). FIG. 24 shows that adsorption of the CAP-oligonucleotide onto NPs significantly reduces its ability to detect the target oligonucleotide.

### Example 21 - Analysis of the TET-miRNA Reaction when the Capture-Oligonucleotide is in Solution Versus Immobilized Via Biotin-Streptavidin to SiO2 NPs

Measurements of enzyme activity were made using a reaction mixture consisting of 20 µl His-Si-BST, 20 µl His-Si-ATPS, 5 µl His-Si-Luciferase, 5 µl luciferin (200 mM), 5 µl of 20X Luciferase buffer (50 mM HEPES, 40 mM KCL, 200 mM MgCl₂), 2 µl APS (30 mM), 1 µl of NP-Cap-Oligo (T2 (SEQ ID NO:7), 100 µM), and 1.8 µl of dCTP/dTTP/dGTP mix (33 mM each). The reaction mixture was added into individual wells of a white 96 well plate containing 1 µl of the target oligonucleotide (R6 (SEQ ID NO:6), 1 µM) and immediately placed into a TECAN plate reader to read the luminescence signal at room temperature for 2000 seconds with 400 msec integration time.

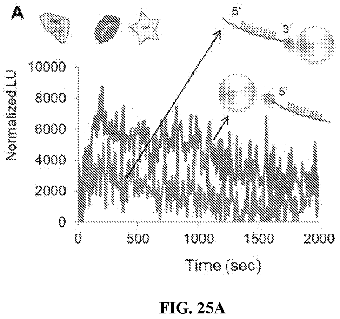

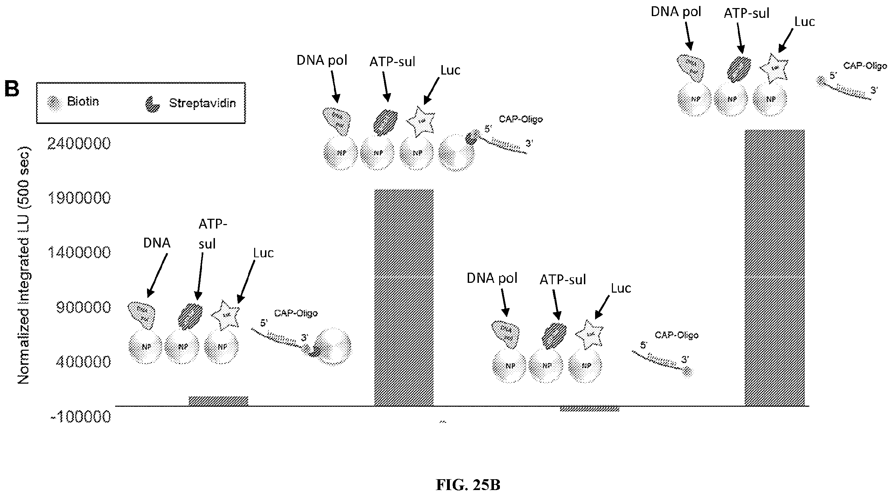

FIG. 25A identifies 2 versions of an immobilizable CAP-oligonucleotide that were generated, where a biotin tag is attached to either 5' or 3' ends (biotinylated oligonucleotides were purchased from IDT, CA, USA). The 3' and 5' biotinylated oligonucleotides were immobilized onto streptavidin coated SiO₂ NPs (500nm, Bangs Laboratory, IN, USA) according to manufacturer instructions, and then spin washed of unbound protein 3 times. TET-mRNA reactions demonstrated low activity using CAP-oligonucleotides tethered to streptavidin coated 500 nm SiO₂ NPs while the enzymes (DNA-Pol, ATPs and Luc) were in solution. FIG. 25B shows that significantly higher activity was obtained when the TET-mRNA enzymes were also immobilized onto NPs (via the Si-tag) in the presence of the 5' biotinylated Cap-oligo; however, the 3' biotinylated CAP-oligo demonstrated very low or no activity.

Although preferred embodiments have been depicted and described in detail herein, it will be apparent to those skilled in the relevant art that various modifications, additions, substitutions, and the like can be made and these are therefore considered to be within the scope of the invention as defined in the claims which follow.

## Claims

1. A method of detecting a target nucleic acid molecule in a sample, said method comprising:
contacting a sample with a capture oligonucleotide molecule complementary to at least a portion of the target nucleic acid molecule so that the capture oligonucleotide molecule hybridizes to a complementary portion of the target nucleotide molecule and forms a double-stranded nucleic acid molecule, wherein said capture oligonucleotide molecule has:
(i) a length of 30-60 bases,
(ii) a 3' tail consisting of 4 to 8 nucleotides,
(iii)a 5' tail,
(iv) a target-specific portion between the 3' tail and the 5' tail,
(v) a deoxy-adenosine phosphate content of 40-50%,
(vi) no deoxy thymidine phosphate in the 3' tail or the 5' tail, and
(vii) the 3' tail and the 5' tail collectively comprising a deoxy-adenosine phosphate content which is 40-50% of said length of 30-60 bases of the capture oligonucleotide molecule;
contacting the double-stranded nucleic acid molecule, a polymerase, and a dNTP mixture to form a polymerase extension mixture, wherein deoxy-adenosine triphosphate is excluded from the dNTP mixture;
subjecting the polymerase extension mixture to conditions under which the target nucleic acid molecule is extended isothermally and releases free phosphates;
producing adenosine triphosphates from the released free phosphates; and
metabolizing the adenosine triphosphates produced from the free phosphates with a luciferase to produce a bioluminescent readout signal, indicating the presence of the target nucleic acid molecule in the sample.

2. The method of claim 1, wherein:
a) the DNA polymerase is coupled to a solid support, optionally is coupled to the solid support with a linker selected from the group consisting of His-Si, His, Si, biotin, streptavidin, Pt, Au, Ag, His-Pt, His-Au, His-Ag, GST, antibody, and epitope tag; and/or
b) the luciferase is coupled to a solid support, optionally wherein the luciferase is coupled to the solid support with a linker selected from the group consisting of His-Si, His, Si, biotin, streptavidin, Pt, Au, Ag, His-Pt, His-Au, His-Ag, GST, antibody, and epitope tag.

3. The method of claim 1 or 2, wherein said producing adenosine triphosphates comprises:
a) subjecting the released free phosphates to a coupled glyceraldehyde 3-phosphate dehydrogenase-phosphoglycerate kinase enzymatic reaction to produce adenosine triphosphate,
optionally wherein said subjecting the released free phosphates to a coupled glyceraldehyde 3-phosphate dehydrogenase-phosphoglycerate kinase enzymatic reaction comprises:
contacting adenosine diphosphate, nicotinamide adenine dinucleotide and glyceraldehyde 3-phosphate to achieve the coupled glyceraldehyde 3-phosphate dehydrogenase-phosphoglycerate kinase enzymatic reaction, optionally wherein the glyceraldehyde 3-phosphate dehydrogenase and the phosphoglycerate kinase are coupled to a solid support; or
b)contacting the released free phosphates with adenosine 5'-phosphosulfate in the presence of adenosine triphosphate sulfurylase to produce adenosine triphosphate, optionally wherein the adenosine triphosphate sulfurylase is coupled to a solid support.

4. The method of any of claims 1-3, wherein the target nucleic acid molecule is:
a) present in the sample in a concentration of less than 10⁻⁵ moles per liter; and/or
b) micro-RNA.

5. The method of claim 1, wherein said subjecting is carried out at a temperature of 0 to 100° C, optionally at a temperature of 25 to 40°C.

6. The method of any of claims 1-5, wherein the polymerase is full length BST DNA polymerase, large fragment BST DNA polymerase, BST 2.0 DNA Polymerase, Klenow fragment (3' to 5' exo), and DNA Polymerase I (large Klenow fragment).

7. The method of any of claims 1-6 further comprising:
quantifying the bioluminescent readout signal to determine the presence or concentration of the target nucleic acid molecule in the sample, optionally
a) wherein the presence of the target nucleic acid molecule in the sample is determined, optionally determined by a procedure comprising:
calculating an initial rate of bioluminescent signal production;
calculating what time period is needed to achieve peak bioluminescence; and
calculating bioluminescent signal peak amplitude or integrated bioluminescent signal from time zero to peak bioluminescence;
b) wherein the concentration of the target nucleic acid molecule in the sample is determined.

8. The method of any of claims 1-7, wherein the sample is selected from the group consisting of blood, urine, cerebrospinal fluid, saliva, tissue, and a synthetic material.

9. The method of any of claims 1-8, wherein the method is carried out in solution.

10. The method of any of claims 1-9, wherein multiple capture oligonucleotide molecules are provided for detecting multiple target nucleic acid molecules.

11. A method of detecting a target nucleic acid molecule in a sample according to claim 1 wherein the DNA polymerase, the luciferase, and the enzyme producing adenosine triphosphates are each coupled to a solid support.

12. The method of claim 11, wherein multiple capture oligonucleotide molecules are provided for detecting multiple target nucleic acid molecules.

13. A kit for detecting a target nucleic acid molecule in a sample, said kit comprising:
a capture oligonucleotide molecule complementary to at least a portion of the target nucleic acid molecule so that the capture oligonucleotide molecule hybridizes to a complementary portion of the target nucleic acid molecule and forms a double-stranded nucleic acid molecule, wherein said capture oligonucleotide molecule has (i) a length of 30-60 bases, (ii) a 3' tail consisting of 4 to 8 nucleotides, (iii) a 5' tail, (iv) a target-specific portion between the 3' tail and the 5' tail, (v) a deoxy-adenosine phosphate content of 40-50%, (vi) no deoxy thymidine phosphate in the 3' tail or the 5' tail, and (vii) the 3' tail and the 5' tail collectively comprising a deoxy-adenosine phosphate content which is 40-50% of that of said length of 30-60 bases of the capture oligonucleotide molecule;
a polymerase coupled to a solid support;
a dNTP mixture, wherein deoxy-adenosine triphosphate is excluded from the dNTP mixture;
an enzyme for producing adenosine triphosphates from released free phosphates coupled to a solid support; and
a luciferase for producing a bioluminescent readout signal, said luciferase coupled to a solid support,
optionally wherein the kit comprises multiple capture oligonucleotide molecules for detecting multiple target nucleic acid molecules.

14. A kit for detecting a target nucleic acid molecule in a sample, said kit comprising:
a capture oligonucleotide molecule complementary to at least a portion of the target nucleic acid molecule so that the capture oligonucleotide molecule hybridizes to a complementary portion of the target nucleic acid molecule and forms a double-stranded nucleic acid molecule, wherein said capture oligonucleotide molecule has (i) a length of 30-60 bases, (ii) a 3'tail consisting of 4 to 8 nucleotides, (iii) a 5' tail, (iv) a target-specific portion between the 3' tail and the 5' tail, (v) a deoxy-adenosine phosphate content of 40-50%, (vi) no deoxy thymidine phosphate in the 3' tail or the 5' tail, and (vii) the 3' tail and the 5' tail collectively comprising a deoxy-adenosine phosphate content which is 40-50% of that of said length of 30-60 bases of the capture oligonucleotide molecule;
a polymerase;
a dNTP mixture, wherein deoxy-adenosine triphosphate is excluded from the dNTP mixture;
an enzyme for producing adenosine triphosphates from released free phosphates; and
a luciferase for producing a bioluminescent readout signal,
optionally wherein the kit comprises multiple capture oligonucleotide molecules for detecting multiple target nucleic acid molecules.

15. Use of a composition in a method of claim 1, said composition comprising:
a capture oligonucleotide molecule, wherein the capture oligonucleotide molecule: has (i) a length of 30-60 bases, (ii) a 3' tail consisting of 4 to 8 nucleotides, (iii) a 5' tail, (iv) a target-specific portion between the 3' tail and the 5' tail, (v) a deoxy-adenosine phosphate content of 40-50%, (vi) no deoxy thymidine phosphate in the 3' tail or the 5' tail, wherein the 3' tail and the 5' tail collectively comprise a deoxy-adenosine phosphate content which is 40-50% of said length of 30-60 bases of the capture oligonucleotide molecule.

## Patentansprüche

1. Verfahren zum Erfassen eines Zielnukleinsäuremoleküls in einer Probe, das Verfahren umfassend:
Kontaktieren einer Probe mit einem Abfangoligonukleotidmolekül, das zu mindestens einem Abschnitt des Zielnukleinsäuremoleküls komplementär ist, sodass das Abfangoligonukleotidmolekül mit einem komplementären Abschnitt des Zielnukleotidmoleküls hybridisiert und ein doppelsträngiges Nukleinsäuremolekül bildet, wobei das Abfangoligonukleotidmolekül Folgendes aufweist:
(i) eine Länge von 30-60 Basen,
(ii) einen 3'-Schwanz, der aus 4 bis 8 Nukleotiden besteht,
(iii) einen 5'-Schwanz,
(iv) einen zielspezifischen Abschnitt zwischen dem 3'-Schwanz und dem 5'-Schwanz,
(v) einen Gehalt an Desoxyadenosinphosphat von 40-50 %,
(vi) kein Desoxythymidinphosphat im 3'- oder 5'-Schwanz, und
(vii) der 3'-Schwanz und der 5'-Schwanz gemeinsam einen Gehalt an Desoxyadenosinphosphat aufweisen, der 40-50 % der genannten Länge von 30-60 Basen des Abfangoligonukleotidmoleküls beträgt;
Kontaktieren des doppelsträngigen Nukleinsäuremoleküls, einer Polymerase und einer dNTP-Mischung, um eine Polymeraseverlängerungsmischung zu bilden, wobei Desoxyadenosintriphosphat von der dNTP-Mischung ausgeschlossen ist;
Unterwerfen der Polymeraseverlängerungsmischung den Bedingungen, unter denen das Zielnukleinsäuremolekül isotherm verlängert wird und freie Phosphate freisetzt;
Herstellen von Adenosintriphosphaten aus den freigesetzten freien Phosphaten; und
Metabolisieren der aus den freien Phosphaten hergestellten Adenosintriphosphate mit einer Luciferase, um ein biolumineszentes Auslesesignal zu erzeugen, das die Anwesenheit des Zielnukleinsäuremoleküls in der Probe anzeigt.

2. Verfahren nach Anspruch 1, wobei:
a) die DNA-Polymerase an einen festen Träger gekoppelt ist, optional mit einem Linker an den festen Träger gekoppelt ist, ausgewählt aus der Gruppe, bestehend aus His-Si, His, Si, Biotin, Streptavidin, Pt, Au, Ag, His-Pt, His-Au, His-Ag, GST, Antikörper und Epitop-Tag; und/oder
b) die Luciferase an einen festen Träger gekoppelt ist, wobei die Luciferase optional mit einem Linker aus der Gruppe ausgewählt ist, bestehend aus His-Si, His, Si, Biotin, Streptavidin, Pt, Au, Ag, His-Pt, His-Au, His-Ag, GST, Antikörper und Epitop-Tag an den festen Träger gekoppelt ist.

3. Verfahren nach Anspruch 1 oder 2, wobei das Herstellen von Adenosintriphosphaten Folgendes umfasst:
a) Unterwerfen der freigesetzten freien Phosphate einer gekoppelten Glyceraldehyd-3-Phosphat-Dehydrogenase-Phosphoglycerat-Kinase-Enzymreaktion, um Adenosintriphosphat zu erzeugen, optional, wobei das Unterwerfen der freigesetzten freien Phosphate einer gekoppelten Glyceraldehyd-3-Phosphat-Dehydrogenase-Phosphoglycerat-Kinase-Enzymreaktion Folgendes umfasst: Kontaktieren von Adenosindiphosphat, Nikotinamid-Adenin-Dinukleotid und Glyceraldehyd-3-Phosphat, um die gekoppelte Glyceraldehyd-3-Phosphat-Dehydrogenase-Phosphoglyceratkinase-Enzymreaktion zu erreichen, wobei optional die Glyceraldehyd-3-Phosphat-Dehydrogenase und die Phosphoglyceratkinase an einen festen Träger gekoppelt sind; oder
b) Kontaktieren der freigesetzten freien Phosphate mit Adenosin-5'-phosphosulfat in Gegenwart von Adenosintriphosphatsulfurylase zur Herstellung von Adenosintriphosphat, wobei die Adenosintriphosphatsulfurylase optional an einen festen Träger gekoppelt ist.

4. Verfahren nach einem der Ansprüche 1-3, wobei das Zielnukleinsäuremolekül Folgendes ist:
a) in der Probe in einer Konzentration von weniger als 10⁻⁵ Mol pro Liter vorhanden; und/oder
b) micro-RNA.

5. Verfahren nach Anspruch 1, wobei das Unterwerfen bei einer Temperatur von 0 bis 100 °C, optional bei einer Temperatur von 25 bis 40 °C, durchgeführt wird.

6. Verfahren nach einem der Ansprüche 1 bis 5, wobei die Polymerase BST-DNA-Polymerase in voller Länge, BST-DNA-Polymerase mit großem Fragment, BST-2.0-DNA-Polymerase, Klenow-Fragment (3'-5'-Exo) und DNA-Polymerase I (großes Klenow-Fragment) ist.

7. Verfahren nach einem der Ansprüche 1 bis 6, umfassend:
Quantifizieren des biolumineszenten Auslesesignals, um das Vorhandensein oder die Konzentration des Zielnukleinsäuremoleküls in der Probe zu bestimmen, optional
a) wobei das Vorhandensein des Zielnukleinsäuremoleküls in der Probe bestimmt wird, optional bestimmt durch ein Verfahren, umfassend:
Berechnen einer anfänglichen Rate der biolumineszenten Signalherstellung;
Berechnen der Zeitspanne, die benötigt wird, um den Spitzenwert der Biolumineszenz zu erreichen; und
Berechnen der Spitzenamplitude des Biolumineszenzsignals oder des integrierten Biolumineszenzsignals vom Zeitpunkt Null bis zum Spitzenwert der Biolumineszenz;
b) wobei die Konzentration des Zielnukleinsäuremoleküls in der Probe bestimmt wird.

8. Verfahren nach einem der Ansprüche 1 bis 7, wobei die Probe ausgewählt ist aus der Gruppe, bestehend aus Blut, Urin, Liquor, Speichel, Gewebe und einem synthetischen Material.

9. Verfahren nach einem der Ansprüche 1 bis 8, wobei das Verfahren in Lösung durchgeführt wird.

10. Verfahren nach einem der Ansprüche 1 bis 9, wobei mehrere Abfangoligonukleotidmoleküle zum Erfassen mehrerer Zielnukleinsäuremoleküle bereitgestellt werden.

11. Verfahren zum Erfassen eines Zielnukleinsäuremoleküls in einer Probe nach Anspruch 1, wobei die DNA-Polymerase, die Luciferase und das Adenosintriphosphate produzierende Enzym jeweils an einen festen Träger gekoppelt sind.

12. Verfahren nach Anspruch 11, wobei mehrere Abfangoligonukleotidmoleküle zum Erfassen mehrerer Zielnukleinsäuremoleküle bereitgestellt werden.

13. Kit zum Erfassen eines Zielnukleinsäuremoleküls in einer Probe, das Kit umfassend:
ein Abfangoligonukleotidmolekül, das zu mindestens einem Abschnitt des Zielnukleinsäuremoleküls komplementär ist, sodass das Abfangoligonukleotidmolekül an einen komplementären Abschnitt des Zielnukleinsäuremoleküls hybridisiert und ein doppelsträngiges Nukleinsäuremolekül bildet, wobei das Abfangoligonukleotidmolekül (i) eine Länge von 30-60 Basen, (ii) einen 3'-Schwanz, bestehend aus 4 bis 8 Nukleotiden, (iii) einen 5'-Schwanz, (iv) einen zielspezifischen Abschnitt zwischen dem 3'-Schwanz und dem 5'-Schwanz, (v) einen Desoxyadenosinphosphatgehalt von 40-50 %, (vi) kein Desoxythymidinphosphat im 3'-Schwanz oder im 5'-Schwanz aufweist, und (vii) der 3'-Schwanz und der 5'-Schwanz umfassen zusammen einen Desoxyadenosinphosphatgehalt, der 40-50 % desjenigen der Länge von 30-60 Basen des Abfangoligonukleotidmoleküls beträgt;
eine Polymerase, die an einen festen Träger gekoppelt ist;
eine dNTP-Mischung, wobei Desoxyadenosintriphosphat von der dNTP-Mischung ausgeschlossen ist;
ein Enzym zur Herstellung von Adenosintriphosphaten aus freigesetzten freien Phosphaten, das an einen festen Träger gekoppelt ist; und
eine Luciferase zur Herstellung eines biolumineszenten Auslesesignals, wobei die Luciferase an einen festen Träger gekoppelt ist,
optional, wobei das Kit mehrere Abfangoligonukleotidmoleküle zum Erfassen mehrerer Zielnukleinsäuremoleküle umfasst.

14. Kit zum Erfassen eines Zielnukleinsäuremoleküls in einer Probe, das Kit umfassend:
ein Abfangoligonukleotidmolekül, das zu mindestens einem Abschnitt des Zielnukleinsäuremoleküls komplementär ist, sodass das Abfangoligonukleotidmolekül an einen komplementären Abschnitt des Zielnukleinsäuremoleküls hybridisiert und ein doppelsträngiges Nukleinsäuremolekül bildet, wobei das Abfangoligonukleotidmolekül (i) eine Länge von 30-60 Basen, (ii) einen 3'-Schwanz, bestehend aus 4 bis 8 Nukleotiden, (iii) einen 5'-Schwanz, (iv) einen zielspezifischen Abschnitt zwischen dem 3'-Schwanz und dem 5'-Schwanz, (v) einen Desoxyadenosinphosphatgehalt von 40-50 %, (vi) kein Desoxythymidinphosphat im 3'-Schwanz oder im 5'-Schwanz aufweist, und (vii) der 3'-Schwanz und der 5'-Schwanz umfassen zusammen einen Desoxyadenosinphosphatgehalt, der 40-50 % desjenigen der Länge von 30-60 Basen des Abfangoligonukleotidmoleküls beträgt;
eine Polymerase;
eine dNTP-Mischung, wobei Desoxyadenosintriphosphat von der dNTP-Mischung ausgeschlossen ist;
ein Enzym zur Herstellung von Adenosintriphosphaten aus freigesetzten freien Phosphaten; und
eine Luciferase zur Herstellung eines biolumineszenten Auslesesignals, wobei das Kit optional mehrere Abfangoligonukleotidmoleküle zum Erfassen mehrerer Zielnukleinsäuremoleküle umfasst.

15. Verwendung einer Zusammensetzung in einem Verfahren nach Anspruch 1, die Zusammensetzung umfassend:
ein Abfangoligonukleotidmolekül, wobei das Abfangoligonukleotidmolekül: (i) eine Länge von 30-60 Basen, (ii) einen 3'-Schwanz, der aus 4 bis 8 Nukleotiden besteht, (iii) einen 5'-Schwanz, (iv) einen zielspezifischen Abschnitt zwischen dem 3'-Schwanz und dem 5'-Schwanz, (v) einen Desoxyadenosinphosphatgehalt von 40-50 %, (vi) kein Desoxythymidinphosphat im 3'-Schwanz oder dem 5'-Schwanz aufweist, wobei der 3'-Schwanz und der 5'-Schwanz zusammen einen Desoxyadenosinphosphatgehalt aufweisen, der 40-50 % der Länge von 30-60 Basen des Abfangoligonukleotidmoleküls beträgt.

## Revendications

1. Procédé de détection d'une molécule d'acide nucléique cible dans un échantillon, ledit procédé comprenant :
la mise en contact d'un échantillon avec une molécule d'oligonucléotide de capture complémentaire à au moins une partie de la molécule d'acide nucléique cible, de sorte que la molécule d'oligonucléotide de capture s'hybride à une partie complémentaire de la molécule de nucléotide cible et forme une molécule d'acide nucléique double brin, dans lequel ladite molécule d'oligonucléotide de capture présentant les caractéristiques suivantes :
(i) une longueur de 30 à 60 bases,
(ii) une queue 3' composée de 4 à 8 nucléotides,
(iii) une queue 5',
(iv) une partie spécifique à la cible située entre la queue de 3' et la queue de 5',
(v) une teneur en désoxy-adénosine phosphate de 40 à 50 %,
(vi) aucune désoxy-thymidine phosphate dans la queue 3' ou la queue 5', et
(vii) la queue 3' et la queue 5' comprenant collectivement une teneur en désoxy-adénosine phosphate qui représente 40 à 50 % de ladite longueur de 30 à 60 bases de la molécule d'oligonucléotide de capture ;
la mise en contact entre la molécule d'acide nucléique double brin, une polymérase et un mélange de dNTP pour former un mélange d'extension de polymérase, dans lequel le désoxy-adénosine triphosphate est exclu du mélange de dNTP ;
la soumission du mélange d'extension de polymérase à des conditions dans lesquelles la molécule d'acide nucléique cible est étendue de manière isotherme et libère des phosphates libres ;
la production des adénosine triphosphates à partir des phosphates libres libérés ; et
la métabolisation des adénosine triphosphates produits à partir des phosphates libres avec une luciférase pour produire un signal de lecture bioluminescent, indiquant la présence de la molécule d'acide nucléique cible dans l'échantillon.

2. Procédé selon la revendication 1, dans lequel :
a) l'ADN polymérase est couplée à un support solide, éventuellement couplée au support solide par un lieur choisi parmi His-Si, His, Si, la biotine, la streptavidine, Pt, Au, Ag, His-Pt, His-Au, His-Ag, GST, un anticorps et une étiquette d'épitope ; et/ou
b) la luciférase est couplée à un support solide, éventuellement dans lequel la luciférase est couplée au support solide avec un lieur choisi parmi le groupe constitué de His-Si, His, Si, biotine, streptavidine, Pt, Au, Ag, His-Pt, His-Au, His-Ag, GST, anticorps et étiquette d'épitope.

3. Procédé selon la revendication 1 ou 2, dans lequel lesdits adénosine triphosphates produits comprennent :
a) la soumission des phosphates libres libérés à une réaction enzymatique couplée glycéraldéhyde 3-phosphate déshydrogénase-phosphoglycérate kinase pour produire de l'adénosine triphosphate, dans lequel la soumission des phosphates libres libérés à une réaction enzymatique couplée glycéraldéhyde 3-phosphate déshydrogénase-phosphoglycérate kinase comprend éventuellement :
la mise en contact de l'adénosine diphosphate, du nicotinamide adénine dinucléotide et du glycéraldéhyde 3-phosphate pour réaliser la réaction enzymatique couplée glycéraldéhyde 3-phosphate déshydrogénase-phosphoglycérate kinase, éventuellement dans lequel la glycéraldéhyde 3-phosphate déshydrogénase et la phosphoglycérate kinase sont couplées à un support solide ; ou
b) la mise en contact des phosphates libres libérés avec l'adénosine 5'-phosphosulfate en présence d'adénosine triphosphate sulfurylase pour produire de l'adénosine triphosphate, éventuellement dans lequel l'adénosine triphosphate sulfurylase est couplée à un support solide.

4. Procédé selon l'une quelconque des revendications 1 à 3, dans lequel la molécule d'acide nucléique cible est :
a) présente dans l'échantillon à une concentration inférieure à 10⁻⁵ moles par litre ; et/ou
b) micro-ARN.

5. Procédé selon la revendication 1, dans lequel ladite soumission est réalisée à une température de 0 à 100 °C, éventuellement à une température de 25 à 40 °C.

6. Procédé selon l'une quelconque des revendications 1 à 5, dans lequel la polymérase est la polymérase d'ADN BST pleine longueur, la polymérase d'ADN BST à grand fragment, la polymérase d'ADN BST 2.0, le fragment Klenow (3' à 5' exo) et la polymérase d'ADN I (grand fragment Klenow).

7. Procédé selon l'une quelconque des revendications 1 à 6, comprenant également :
la quantification du signal de lecture bioluminescent pour déterminer la présence ou la concentration de la molécule d'acide nucléique cible dans l'échantillon, éventuellement
a) dans lequel la présence de la molécule d'acide nucléique cible dans l'échantillon est déterminée, éventuellement par une procédure comprenant :
le calcul d'un taux initial de production de signal bioluminescent ;
le calcul de la période de temps nécessaire pour atteindre la bioluminescence maximale ; et
le calcul de l'amplitude maximale du signal bioluminescent ou du signal bioluminescent intégré du temps zéro à la bioluminescence maximale ;
b) dans lequel la concentration de la molécule d'acide nucléique cible dans l'échantillon est déterminée.

8. Procédé selon l'une quelconque des revendications 1 à 7, dans lequel l'échantillon est choisi parmi le groupe constitué de sang, d'urine, de liquide céphalo-rachidien, de salive, de tissu et d'un matériau synthétique.

9. Procédé selon l'une quelconque des revendications 1 à 8, dans lequel le procédé est mis en œuvre en solution.

10. Procédé selon l'une quelconque des revendications 1 à 9, dans lequel plusieurs molécules d'oligonucléotides de capture sont fournies pour détecter plusieurs molécules d'acide nucléique cibles.

11. Procédé de détection d'une molécule d'acide nucléique cible dans un échantillon selon la revendication 1
dans lequel l'ADN polymérase, la
luciférase et l'enzyme produisant des adénosine triphosphates sont chacune fixées à un support solide.

12. Procédé selon la revendication 11, dans lequel plusieurs molécules d'oligonucléotides de capture sont fournies pour détecter plusieurs molécules d'acide nucléique cibles.

13. Kit pour la détection d'une molécule d'acide nucléique cible dans un échantillon, ledit kit comprenant :
une molécule d'oligonucléotide de capture complémentaire à au moins une partie de la molécule d'acide nucléique cible, de sorte que la molécule d'oligonucléotide de capture s'hybride à une partie complémentaire de la molécule d'acide nucléique cible et forme une molécule d'acide nucléique double brin, dans lequel ladite molécule d'oligonucléotide de capture a (i) une longueur de 30 à 60 bases, (ii) une queue 3' composée de 4 à 8 nucléotides, (iii) une queue 5', (iv) une partie spécifique à la cible entre la queue 3' et la queue 5', (v) une teneur en désoxy-adénosine phosphate de 40 à 50 %, (vi) aucune désoxy-thymidine phosphate dans la queue 3' ou la queue 5', et (vii) la queue 3' et la queue 5' comprenant collectivement une teneur en désoxy-adénosine phosphate qui est de 40 à 50 % de celle de ladite longueur de 30 à 60 bases de la molécule d'oligonucléotide de capture ;
une polymérase couplée à un support solide ;
un mélange de dNTP, dans lequel le désoxy-adénosine triphosphate est exclu du mélange de dNTP ;
une enzyme permettant de produire des adénosine triphosphates à partir de phosphates libres libérés, fixée à un support solide ; et
une luciférase pour produire un signal de lecture bioluminescent, ladite luciférase étant couplée à un support solide,
éventuellement dans lequel le kit comprend plusieurs molécules d'oligonucléotides de capture pour la détection de plusieurs molécules d'acide nucléique cibles.

14. Kit pour la détection d'une molécule d'acide nucléique cible dans un échantillon, ledit kit comprenant :
une molécule d'oligonucléotide de capture complémentaire à au moins une partie de la molécule d'acide nucléique cible, de sorte que la molécule d'oligonucléotide de capture s'hybride à une partie complémentaire de la molécule d'acide nucléique cible et forme une molécule d'acide nucléique double brin, dans lequel ladite molécule d'oligonucléotide de capture a (i) une longueur de 30 à 60 bases, (ii) une queue 3' composée de 4 à 8 nucléotides, (iii) une queue 5', (iv) une partie spécifique à la cible entre la queue 3' et la queue 5', (v) une teneur en désoxy-adénosine phosphate de 40 à 50 %, (vi) aucune désoxy-thymidine phosphate dans la queue 3' ou la queue 5', et (vii) la queue 3' et la queue 5' comprenant collectivement une teneur en désoxy-adénosine phosphate qui est de 40 à 50 % de celle de ladite longueur de 30 à 60 bases de la molécule d'oligonucléotide de capture ;
une polymérase ;
un mélange de dNTP, dans lequel le désoxy-adénosine triphosphate est exclu du mélange de dNTP ;
une enzyme permettant de produire des adénosine triphosphates à partir de phosphates libres libérés ; et
une luciférase pour produire un signal de lecture bioluminescent, dans lequel le kit comprend éventuellement plusieurs molécules d'oligonucléotides de capture pour détecter plusieurs molécules d'acide nucléique cibles.

15. Utilisation d'une composition dans un procédé selon la revendication 1, ladite composition comprenant :
une molécule d'oligonucléotide de capture, dans laquelle la molécule d'oligonucléotide de capture : a (i) une longueur de 30 à 60 bases, (ii) une queue 3' constituée de 4 à 8 nucléotides, (iii) une queue 5', (iv) une partie spécifique à la cible entre la queue 3' et la queue 5', (v) une teneur en désoxy-adénosine phosphate de 40 à 50 %, (vi) aucune désoxy-thymidine phosphate dans la queue 3' ou la queue 5', dans laquelle la queue 3' et la queue 5' comprennent collectivement une teneur en désoxy-adénosine phosphate qui est de 40 à 50 % de ladite longueur de 30 à 60 bases de la molécule d'oligonucléotide de capture.
